# EUROPEAN PATENT APPLICATION

(11) **EP 3 944 257 A1**
(43) Date of publication of application: **26.01.2022**
(21) Application number: 21187036.5
(22) Date of filing: 21.07.2021
(51) Int. Cl.: G16H 50/20, A61B 5/00, A61B 5/11, A61B 5/12, A61B 5/1455, A61B 5/16, G06N 20/00

(54) **AI (ARTIFICIAL INTELLIGENCE) BASED DEVICE AND METHOD FOR PROVIDING BRAIN INFORMATION**

(30) Priority: 22.07.2020 KR 20200091205; 22.07.2020 KR 20200091206
(71) Applicant: Actibrain Bio, Inc., Seoul 06112 (KR)
(72) Inventor: KIM, Sung Yeun, 06112 Seoul (KR)
(74) Representative: ZBM Patents - Zea, Barlocci & Markvardsen

(57) **Abstract**

The present invention relates to an AI (Artificial Intelligence) based method for providing brain information comprising: a step 1 in which a brain signal obtaining portion of a brain signal measuring portion irradiates near-infrared ray to user's brain and detects light penetrating a cerebral cortex of the brain; a step 2 in which a brain signal processing portion of the brain signal measuring portion determines a level of oxygenation of hemoglobin in blood flow of the user's brain on the basis of the detected light; a step 3 in which a brain signal analyzing portion of the brain signal measuring portion extracts at least one brain activation area from a plurality of activated areas of the user's brain on the basis of the determined level of oxygenation of hemoglobin; and a step 4 in which a diagnosing portion determines a state of the user's brain on the basis of the at least one brain activation area extracted successively for a predetermined period of time, wherein a signal collecting operation of the brain signal obtaining portion in the step 1 is performed in a state that the user is moving.

## Description

### CROSS-REFERENCE TO RELATED APPLICATIONS

The present application claims priority to Korean Patent Application Nos. 10-2020-0091205, filed on July 22, 2020 and 10-2020-0091206, filed on July 22, 2020, the entire contents of which are incorporated herein by reference.

### BACKGROUND

### Technical Field

The present invention relates to an AI (Artificial Intelligence) based device and method for providing brain information, more particularly to an AI based method for providing brain information, which collects signals related with the brain of a user in a state of activity and determines the state of the user's brain therefrom and is capable of providing feedback on the brain information.

### Description of the Related Art

According to people's behaviors and circumstances, a brain changes circuits newly and creates either new functions or cells to be grown. Further, the function of brain areas may be changed according to what people thinks.

As a part of the brain areas, the hippocampus that is involved in learning and memory formation performs neurogenesis that forms neurons newly, this neurogenesis occurring the most actively in the hippocampus. The next most active brain area to the hippocampus in the neurogenesis is an olfactory related area, the brain creates a new neuron to distinguish smell when taking a new smell.

However, all nerve fibers including brain cells become to extinction as time goes on rather than long-time existence. Since the extinction of cells means the senescence or degeneration thereof, cell generation and nerve development should be activated rather than the extinction. Accordingly, in order to activate the nerve fibers, particularly brain cells, it is very crucial to find out factors affecting the rate of neurogenesis and the life span of neurons.

When activation means to activate the brain cell generation, neurogenesis, neurogenesis rate and neuron life span, prior-used methods to activate the brain include exercise, foods, stimulants, music, meditation, etc. However, recently neurologists found out the aforementioned factors, these factors adopted to the brain activation to develop methods for improving brain functions.

In the meantime, when testing or diagnosing brain functions, used are various ways such as computer tomography (CT), magnetic resonance imaging (MRI), proton emission tomography (PET), electroencephalograph (EEG), magnetoencephalography (MEG), functional magnetic resonance imaging (FMRI), etc.

### Prior Art document

### Patent Document

(Patent Document 1) Korean Patent No. 10-1754291 (July 06, 2017)
(Patent Document 2) Korean Patent Application Publication No. 10-2016-0058812 (May 25, 2016)
(Patent Document 3) Korean Patent No. 10-1768393 (August 17, 2017)
(Patent Document 4) Korean Patent No. 10-1295187 (August 9, 2013)

### SUMMARY OF THE INVENTION

The present invention aims to provide an AI (Artificial Intelligence) based method for providing brain information.

In particular, the present invention aims to provide an AI based method for providing brain information which collects signals related with the brain of a user in a state of activity and determines the state of the user's brain therefrom and is capable of feedback on the brain information.

Further, the present invention aims to provide a system and method for providing brain information, the system comprising: a brain signal measuring portion that includes a brain signal obtaining portion which irradiates near-infrared ray to user's brain and detects light penetrating the cerebral cortex of the brain, a brain signal processing portion which determines a level of oxygenation of hemoglobin in blood flow of the user's brain on the basis of the detected light and a brain signal analyzing portion which extract at least one brain activation area from a plurality of areas of the user's brain on the basis of the determined level of oxygenation of hemoglobin; and a diagnosing portion that determines the state of the user's brain on the basis of the at least one brain activation area which was extracted successively for a predetermined period of time.

Further, the present invention aims to provide the system and method for providing brain information, the system including a brain signal stimulating portion for stimulating the user's brain.

Further, the present invention aims to provide the system and method for providing brain information, the system determining the state of the user's brain more precisely by additionally using an auditory information collecting portion that collects the user's auditory information, a gait information collecting portion that collects the user's gait information, an electrocardiogram information collecting portion that collects the user's electrocardiogram information, a sleep information collecting portion that collects the user's sleep information, a attention information collecting portion that collects the user's attention information, a non-contact typed emotional change information collecting portion that collects the user's face landmark masking data on the basis of the user's eye tracking to collect the user's emotional change information in a non-contact way, a non-contact typed emotional change information collecting portion that collects the user's emotional change information on the basis of the user's speech change, etc.

Further, the present invention aims to provide the system and method for providing brain information, the system determining the state of the user's brain more precisely by additionally using an imaging collecting portion that collects medical imaging related to the user and thus using the brain activation area, body information related to the user and the collected medical imaging related to the user together.

Further, the present invention aims to provide the system and method for providing brain information, the system comprising a diagnosing portion that is capable of determining brain diseases including dementia, Parkinson's disease, cerebral stroke, Epilepsy, brain tumor and development disorder, and a management portion that provides information for improving brain state, corresponding to the user's brain state determined by the diagnosing portion.

Further, the present invention aims to provide the system and method for providing brain information, the system further using a brain signal stimulating portion to stimulate the user's brain when the user, by applying preset references, is suspected of at least one of dementia, Parkinson's disease, cerebral stroke, Epilepsy, brain tumor and development disorder within a certain period of time, on the basis of the user's brain state.

Meanwhile, technical aims to be achieved in the present invention are not limited to the aforementioned objects, and other not-mentioned technical aims will be obviously understood by those skilled in the art from the description below.

According to an aspect of the present invention, provide is an AI (Artificial Intelligence) based method for providing brain information including: a step 1 in which a brain signal obtaining portion of a brain signal measuring portion irradiates near-infrared ray to user's brain and detects light penetrating a cerebral cortex of the brain; a step 2 in which a brain signal processing portion of the brain signal measuring portion determines a level of oxygenation of hemoglobin in blood flow of the user's brain on the basis of the detected light; a step 3 in which a brain signal analyzing portion of the brain signal measuring portion extracts at least one brain activation area from a plurality of activated areas of the user's brain on the basis of the determined level of oxygenation of hemoglobin; and a step 4 in which a diagnosing portion determines a state of the user's brain on the basis of the at least one brain activation area extracted successively for a predetermined period of time, wherein a signal collecting operation of the brain signal obtaining portion in the step 1 is performed in a state that the user is moving. Further, in the step 2, on the basis of the detected light, oxygenated hemoglobin (Oxy Hb) concentration and deoxygenated hemoglobin (Deoxy Hb) concentration in blood flow of the user may be extracted to determine a level of oxygenation of hemoglobin.

Further, in the at least one brain activation area, oxygen transferred by the Oxy Hb in the blood flow may be consumed to decrease the Oxy Hb concentration and to increase the Deoxy Hb concentration corresponding to the decreased Oxy Hb concentration.

Further, the Oxy Hb concentration and the Deoxy Hb concentration may have an optical property that changes in a visible-ray area and a near-infrared area and in the steps 2, the brain signal measuring portion may collect the signal through functional Near-infrared Spectroscopy (fNIRS).

Further, in the step 3, the brain signal analyzing portion may divide the user's brain area into multiple areas and may determine whether or not each of the divided brain areas is changed to the brain activation area by a predetermined time unit or whether or not the brain activation area is changed to an inactivation area.

Further, in the step 4, the diagnosing portion, with respect to the predetermined time unit, may use a brain activation change in each of a plurality of divided brain areas to determine the user's brain state.

Further, between the step 3 and the step 4, the method may further include a step in which a display portion of the brain signal measuring portion displays the brain activation change in each of the plurality of the divided brain areas according to the time.

Further, in the step 1, the brain signal measuring portion may be a full cover type which covers the whole of the user's head, alternatively a front type which is adhered closely to a front part of the user's head.

Further, prior to the step 1, the method may further include a step 0.5 in which for a signal collecting operation of the brain signal measuring portion, a brain signal stimulating portion stimulates the users' brain.

Further, the step 0.5 may include: a step (a) in which a brain activating device of the brain signal stimulating portion applies multi-dimensional environmental stimulation to reinforce an activity of a pre-targeted brain area within a predetermined space intensively and to activate a function of the brain area; and a step (b) in which a management server controls an operation of the brain activating device to apply a multi-dimensional environmental stimulation to the pre-targeted brain area within the space.

Further, the space may be comparted into a plurality of spaces, and the step (a) may include: a step in which a media portion implements a media art corresponding to a concept to be created in each of the plurality of spaces; a step in which an acoustic portion outputs sound; a step in which a lighting portion irradiates light; and a step in which a scent transferring portion transfers a scent to the user.

Further, in the step (b), the management server may include database in which a plurality of media and acoustic information corresponding to a concept of each of the plurality of spaces and a program for controlling the operation of the brain activating device are stored, may select at least one of a plurality of media and acoustic information files and programs corresponding to the concept of each of the spaces from various kinds of the information and programs stored in the database, and may control the operation of the brain activating device allowing changing according to the selected information and program.

Further, the media portion may include a plurality of media modules which implement a media art corresponding to the concept of each of the spaces, each of the media modules including a projector or a display panel which projects the media art including a photo or a video, the acoustic portion including a plurality of acoustic modules which output sound corresponding to the concept of each of the spaces, and the each of the acoustic modules including a speaker and an amplifier which output sound including natural white noise.

Further, the lighting portion may include a plurality of lighting modules which irradiate light corresponding to the concept of each of the spaces, each of the lighting modules composed of a combination of one or two of a laser generator, an LED and a neon lighting, the scene transferring portion including a plurality of transferring modules which transfer scent generated from a natural object disposed in each of the spaces to the user, and each of the transferring modules including an air blower which blows air to transfer the scent generated from a natural object disposed in each of the spaces or a copy scent thereof to the user of each of the spaces.

Further, following the step (b), the step 0.5 may further include a step (c) in which, on the basis of manager's operation, a manager' terminal that controls operation of the brain activating device selects at least one of the plurality of the information files and programs stored in the database of the management server for each of the modules to be installed in each of the spaces, and then changes media, sounds, lightings, etc., according to the selected information files and programs periodically or unperiodically.

Further, following the step (b), the step 0.5 may further include a step (c) in which a user's terminal that is capable of communicating with the management server and inputs the user's operation instruction inputs the user's information including age, gender, preference and mental state from the user and then transfers the inputted user's information to the management server; and a step (d) in which the management server determines the user's age, gender, preference and mental state and performs controlling to provide media, sounds and lightings corresponding to a personalized concept.

Further, between the step 3 and the step 4, the method may further include a step 3-5 in which a user data collecting portion collects body information related to the user.

Further, in the step 3-5, an auditory information collecting portion of the user data collecting portion may collect the user's auditory data. In the step 4, the diagnosing portion may determine a level of the user's hearing damage on the basis of the collected auditory information. Further, in the step 4, the diagnosing portion may determine the user's brain state using the at least one the brain activation area together with the level of user's hearing damage.

Further, when the user's hearing damage results from hearing loss, the diagnosing portion determines the user's brain state by additionally using the body information related to the user, an average value of pure-tone thresholds in both of the user's ears, a level of the user's hearing loss and information related to the auditory information collecting portion.

Further, when the user's hearing damage results from Tinnitus, the diagnosing portion determines the user's brain state by additionally using the body information related to the user, an average value of pure-tone thresholds in both of the user's ears, a level of the user's hearing loss and information related to the auditory information collecting portion.

Further, in the step 1, a signal collecting operation of the brain signal measuring portion may be performed in a state that the user is moving.

Further, in the step 3-5, the user data collecting portion includes at least one of a gait information collecting portion that collects the user's gait information collects the user's gait information, a stress collecting portion that collect the user's stress information, an electrocardiogram information collecting portion that collects the user's electrocardiogram information, a sleep information collecting portion that collects the user's sleep information and a attention information collecting portion that collects the user's attention information. In the step 4, the diagnosing portion may determine, on the basis of the collected information, at least one of the user's gait pattern, a level of the user's stress, and the user's electrocardiogram change, sleep type, attention change. In the step 4, the diagnosing portion may determine at least one of a level of the user's hearing damage, the user's gait pattern, a level of the user's stress, and the user's electrocardiogram change, sleep type, attention change together with the at least one brain activation area.

Further, the signal collecting operation of the brain signal measuring portion may be performed in a circumstance applied with at least one of states in which the user's gait, the user's stress, the user's electrocardiogram, the user's sleep condition and the user's attention are changing, while the user is moving.

Further, in the step 3-5, the user data collecting portion further includes a non-contact typed emotional change information collecting portion that collects the user's face landmark masking data on the basis of the user's eye tracking to collect the user's emotional change information in a non-contact way, and in the step 4, the diagnosing portion may determine the user's brain state using the at least one brain activation area together with the user's emotional change information.

Further, in the step 3-5, the user data collecting portion further includes a non-contact typed emotional change information collecting portion that collects the user's emotional change information on the basis of the user's speech change, and in the step 4, the diagnosing portion may determine the user's brain state using the at least one brain activation area together with the user's emotional change information.

Further, the method further includes, between the step 3.5 and the step 4, a step 3.7 in which an imaging collecting portion collects medical imaging related to the user, wherein in the step 4, the diagnosing portion may determine the state of the user's brain using the at least one brain activation area, body information related to the user and the collected medical imaging related to the user together.

Further, the imaging collection portion of the step 3.7 may include: an MRI imaging scan obtaining portion that collects an MRI imaging scan related to the user; a CT imaging scan obtaining portion that collects a CT imaging scan related to the user; and an fMRI imaging scan obtaining portion that collects an fMRI imaging scan related to the user.

Further, in the step 4, the diagnosing portion may determine the user's brain disease, the brain disease including dementia, mild cognitive impairment (MCI), Parkinson's disease, depression, cerebral stroke, Epilepsy, brain tumor and development disorder, and following the step 4, the method may further include a step 5 in which a management portion provides information for improving the user's brain state, corresponding to the user's brain state determined by the diagnosing portion.

Further, when the user, by applying preset references, is suspected of at least one of dementia, MCI, Parkinson's disease, cerebral stroke, Epilepsy, brain tumor and development disorder within a certain period of time, on the basis of the user's brain state, the step 5 may further include a step in which a brain signal stimulating portion stimulates the user's brain for improving the user's brain state according to controlling of the management portion.

### Advantageous effects

As described above, the present invention is capable of providing an AI based method for providing brain information which collects signals related with the brain of a user in a state of activity and determines the state of the user's brain therefrom and is capable of providing feedback on the brain information.

Further, the present invention is capable of providing a system and method for providing brain information, the system comprising: a brain signal measuring portion that includes a brain signal obtaining portion which irradiates near-infrared ray to user's brain and detects light penetrating the cerebral cortex of the brain, a brain signal processing portion which determines a level of oxygenation of hemoglobin in blood flow of the user's brain on the basis of the detected light and a brain signal analyzing portion which extract at least one brain activation area from a plurality of areas of the user's brain on the basis of the determined level of oxygenation of hemoglobin; and a diagnosing portion that determines the state of the user's brain on the basis of the at least one brain activation area which was extracted successively for a predetermined period of time.

Further, the present invention is capable of providing the system and method for providing brain information, the system including a brain signal stimulating portion for stimulating the user's brain.

Further, the present invention is capable of providing the system and method for providing brain information, the system determining the state of the user's brain more precisely by additionally using an auditory information collecting portion that collects the user's auditory information, a gait information collecting portion that collects the user's gait information, an electrocardiogram information collecting portion that collects the user's electrocardiogram information, a sleep information collecting portion that collects the user's sleep information, a attention information collecting portion that collects the user's attention information, a non-contact typed emotional change information collecting portion that collects the user's face landmark masking data on the basis of the user's eye tracking to collect the user's emotional change information in a non-contact way, a non-contact typed emotional change information collecting portion that collects the user's emotional change information on the basis of the user's speech change, etc.

Further, the present invention is capable of providing the system and method for providing brain information, the system determining the state of the user's brain more precisely by additionally using an imaging collecting portion that collects medical imaging related to the user and thus using the brain activation area, body information related to the user and the collected medical imaging related to the user together.

Further, the present invention is capable of providing the system and method for providing brain information, the system comprising a diagnosing portion that is capable of determining brain diseases including dementia, Parkinson's disease, cerebral stroke, Epilepsy, brain tumor and development disorder, and a management portion that provides information for improving brain state, corresponding to the user's brain state determined by the diagnosing portion.

Further, the present invention is capable of providing the system and method for providing brain information, the system further using a brain signal stimulating portion to stimulate the user's brain when the user, by applying preset references, is suspected of at least one of dementia, Parkinson's disease, cerebral stroke, Epilepsy, brain tumor and development disorder within a certain period of time, on the basis of the user's brain state.

Further, the present invention is capable of being used as a result of the study on degenerative brain diseases, thereby developing and utilizing a non-invasive diagnosis and treatment monitoring system through designing of brain imaging and cognitive ability measurement tests; preparing a base for building brain imaging database and sharing the database through brain structure imaging and brain function imaging tests; and providing the optimum treatment method through examination of a treatment mechanism based on deep-learning analysis.

Further, the present invention is capable of being used as a medical instrument for the optical treatment and diagnosis of degenerative brain disease, thereby implementing prototype of a medical instrument for commercialization of a treatment platform of the degenerative brain diseases; commercializing the medical instrument by cooperating with enterprises on the basis of the study result; and maximizing utilization of the medical instrument by liaison with local dementia care centers.

Furthermore, the present invention is capable of ensuring a high quality AI data set by preparing a standard guideline for each file of AI learning database constructions and verifying quality step by step; of providing basic data essential for AI technology development; and of building a virtuous cycle of ecosystem in private enterprises, which extends and opens the data autonomously according to AI technology development.

Further, the present invention is capable of leading the field of brain science by verifying treatment relation between the degenerative brain disease and hearing loss and of ensuring competitiveness in the field of advanced medical imaging analysis through studying deep-learning method of brain imaging data.

Further, the present invention is capable of ensuring a new distinctive technique for the non-invasive typed early diagnosis method of the degenerative brain disease and of developing a monitoring system of the non-invasive diagnosis and treatment of brain disease on the basis of brain imaging and cognitive ability.

Further, the present invention is capable of contributing to the solution of social problems through reducing dementia related deathrate, increasing cure rate and reducing treatment cost, and to the increment of productivity resulting from increment in the elderly people's social participation.

Further, the present invention is capable of identifying a state of brain health through the prevention and diagnosis of brain diseases when babies or children have the brain diseases including cerebral stroke, Epilepsy, brain tumor and development disorder or are suspected to have symptoms thereof.

Further, the present invention is capable of helping healthy brain development by increasing user's experience value and avoiding uses of sleeping drugs or anesthetic drugs as applying wearable typed and child friendly designed products when measuring brain signal pathway.

Further, the present invention is capable of providing, space for supporting non-contact learning and emotional labor fields, an emotion sharing AI service, that is, a human-centered AI service which utilizes the construction of a multimodal emotional data network and the N-dimensional emotion mapping.

In particular, the present invention is capable of recognizing user's social emotion; providing the construction of an emotional data network and a personalized emotional AI service; and being utilized for supporting non-contact learning and industrial work sites.

Meanwhile, the effects to be achieved by the present disclosure are not limited to the aforementioned effects and other effects, which are not mentioned above, will be apparent to those skilled in the art from the following description.

### BRIEF DESCRIPTION OF THE DRAWINGS

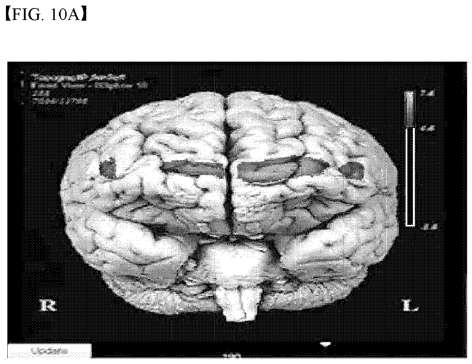

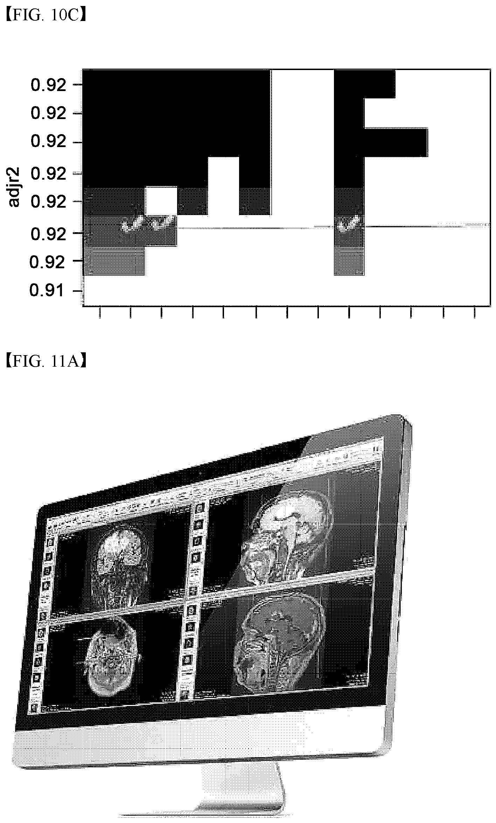

The accompanying drawings in the specification illustrate an embodiment of the present invention. The technical essence of the present disclosure will be more clearly understood from the following detailed description taken in conjunction with the accompanying drawings. Therefore, the present invention will not be interpreted to be limited to the drawings in which:
FIG. 1 is a block diagram showing an Artificial Intelligence (AI) based device for providing brain information.
FIG. 2 is a block diagram of a brain signal measuring portion that is a configurational element of the AI based device for providing brain information.
FIG. 3 is a view for explaining the operation of the brain signal measuring portion.
Each of FIG. 4A and 5B shows one example of a type of the brain signal measuring portion.
Each of FIG. 5A and FIG. 5B shows a view for explaining functional near-infrared spectroscopy to be adopted to the present invention.
Each of FIG. 6A and FIG. 6B is a flowchart for explaining a measuring operation of the brain signal measuring portion.

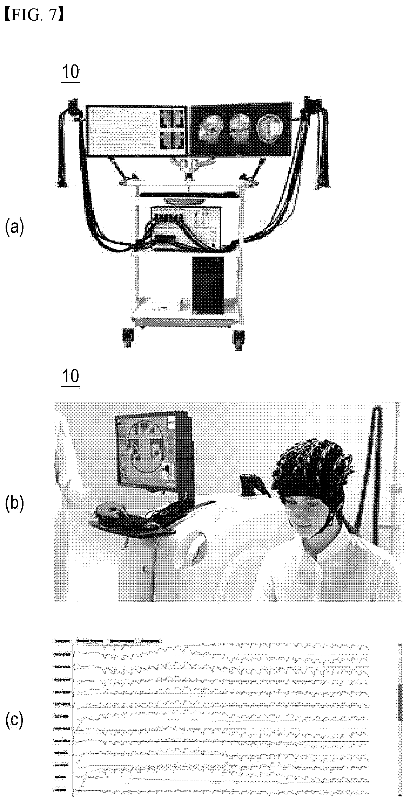

FIG. 7 shows one example of a whole system of the brain signal measuring portion.

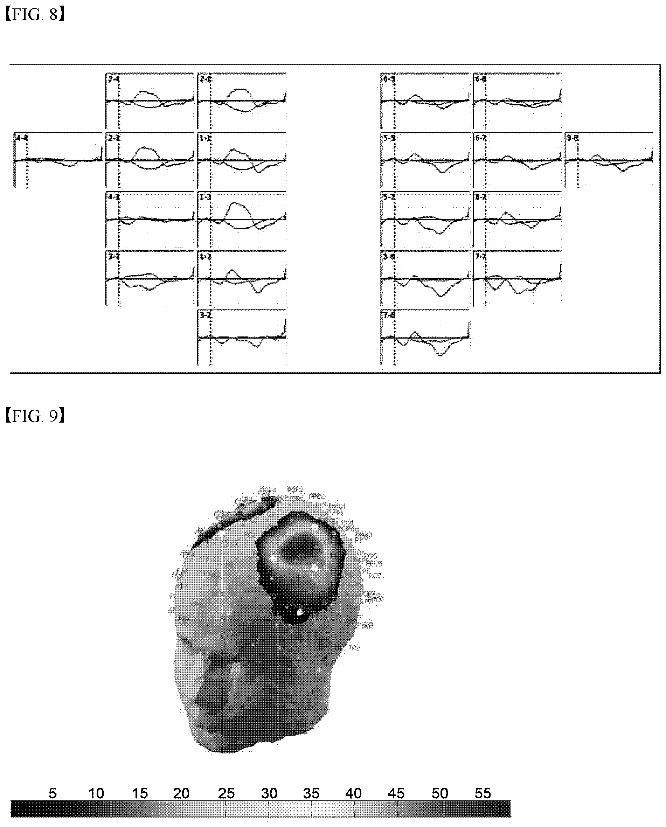

FIG. 8 shows one example of a signal measured by the brain signal measuring portion.
FIG. 9 shows one example visualizing and representing the signal measured by the brain signal measuring portion as brain related information.
Each FIG. 10A to FIG. 10C shows one example of the signal measured by the brain signal measuring portion, visualized information and processed information.
Each of FIG. 11A to FIG. 11C shows one example of an authoring tool for the operation management of AI learning data.

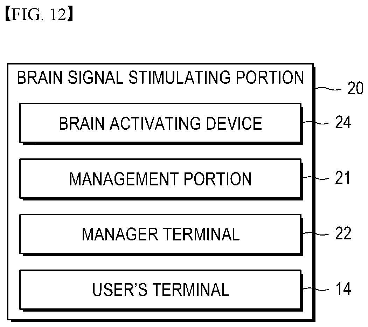

FIG. 12 is a block diagram of a brain signal stimulating portion, that is, a configurational element of the AI based device for providing brain information.

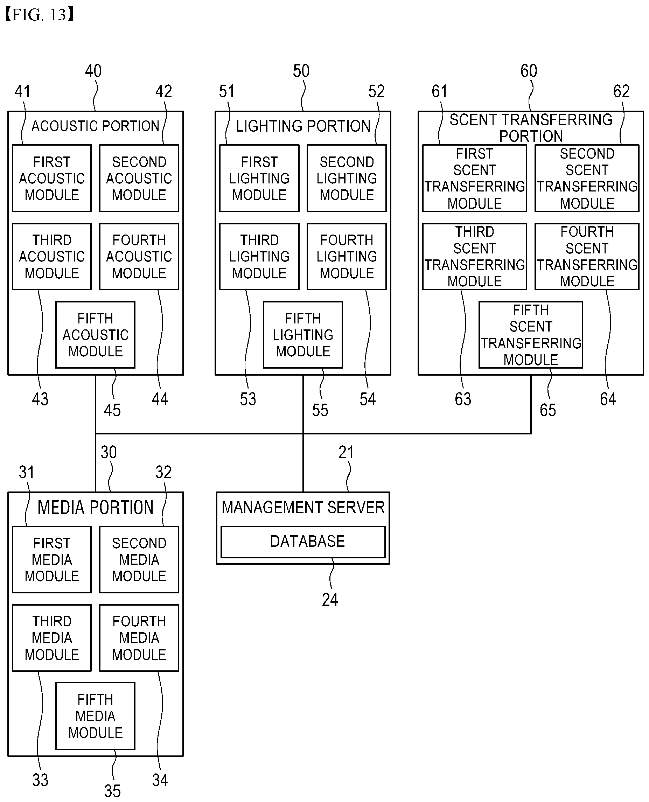

FIG. 13 is a block diagram of a brain activating device of the brain signal stimulating portion.

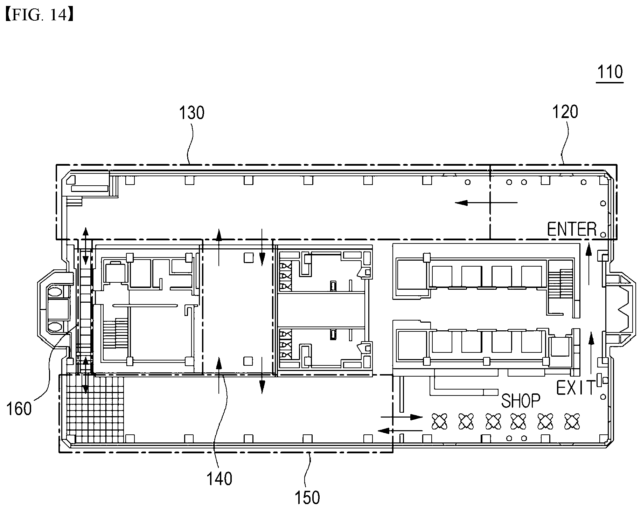

FIG. 14 shows one example building the brain signal stimulating portion spatially.

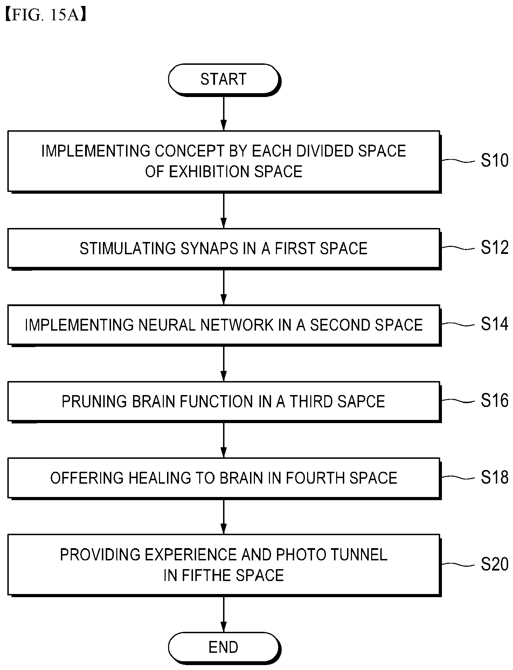

FIG. 15 shows one example of a flowchart explaining a brain activating method of the brain signal stimulating portion.

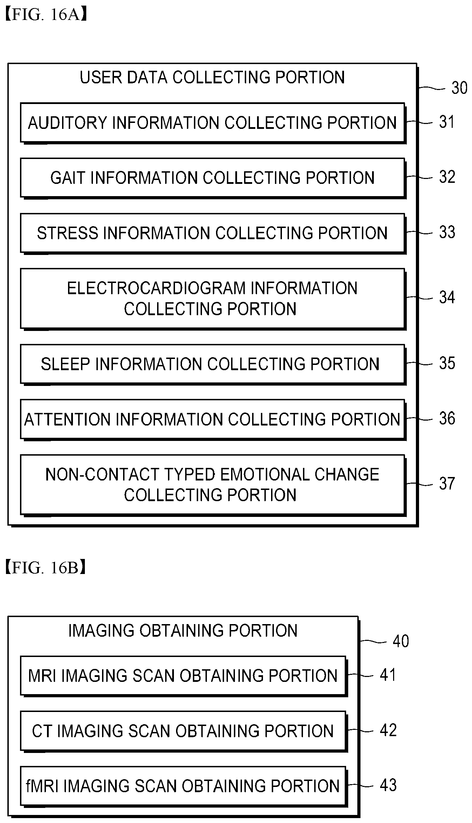

FIG. 16A and FIG. 16B show block diagrams of a user data collecting portion and an imaging obtaining portion.

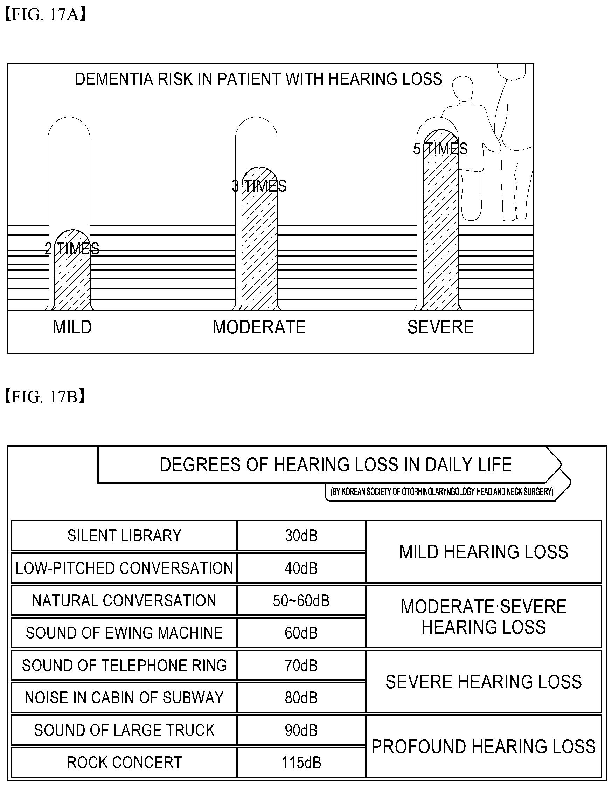

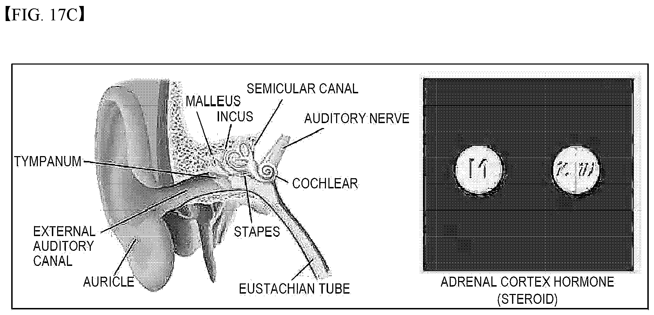

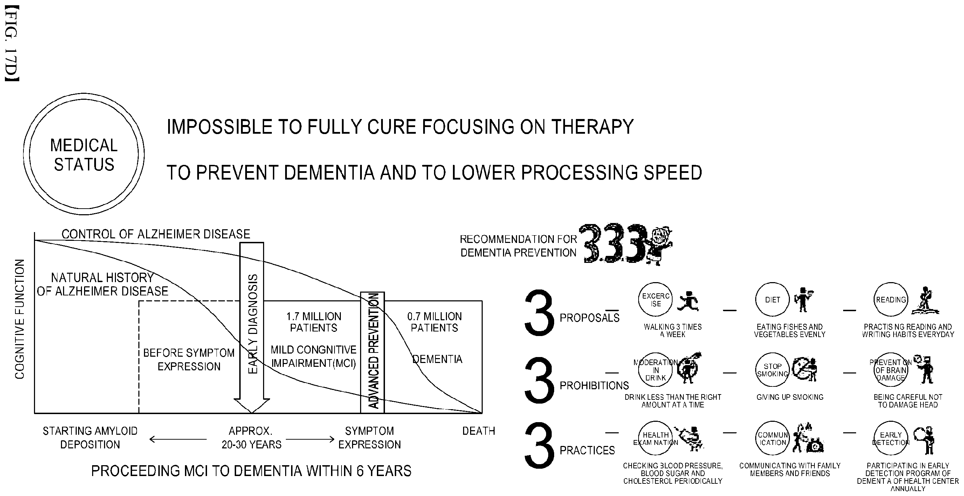

FIG. 17A to FIG. 17D are views explaining correlation between hearing loss and dementia.

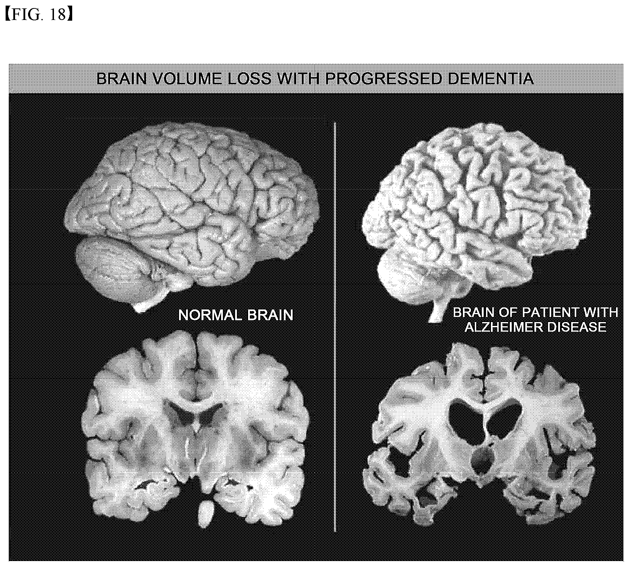

FIG. 18 is a view explaining brain volume loss resulting from Alzheimer's disease.

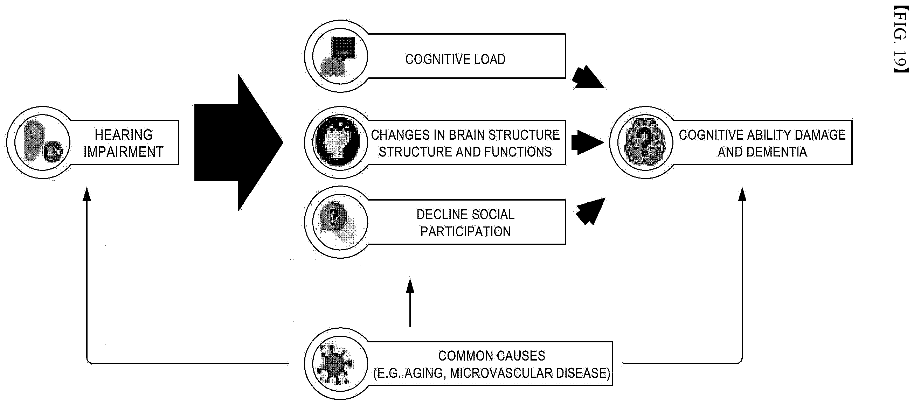

FIG. 19 is a view explaining correlation between cognitive function damage resulting from hearing impairment and dementia.

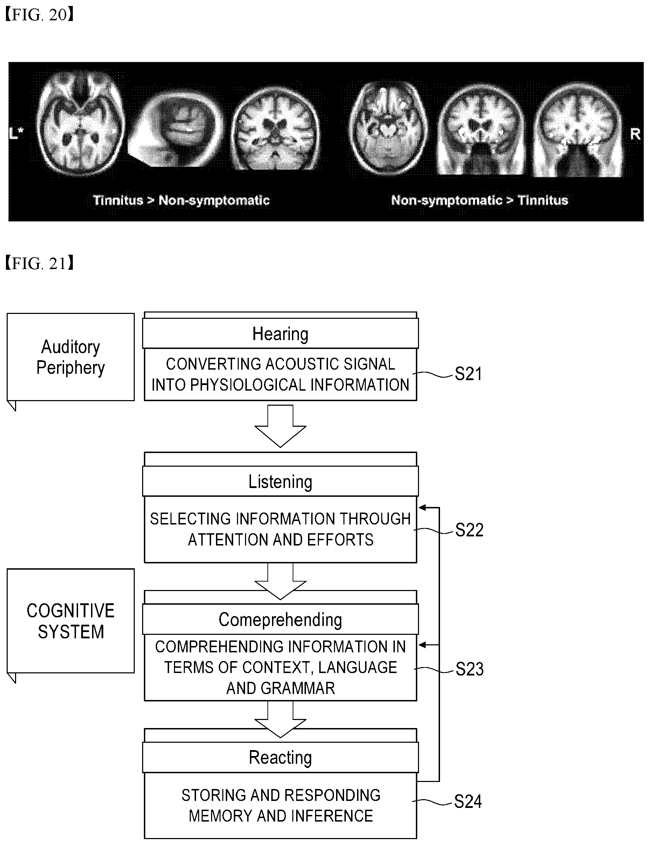

FIG. 20 is a view explaining correlation between hearing loss (tinnitus) and Alzheimer's disease (dementia).
FIG. 21 is a view explaining correlation between hearing and cognitive impairment.

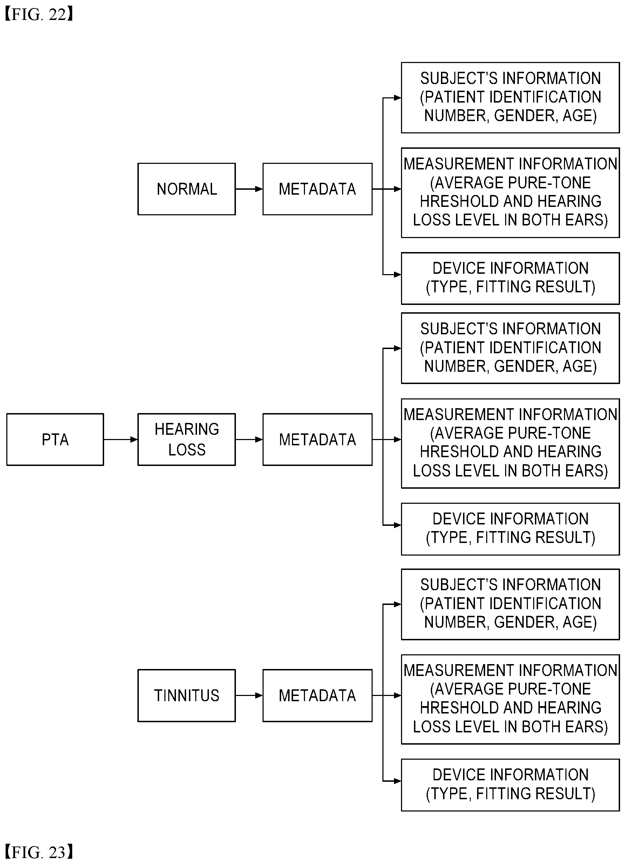

FIG. 22 is a view explaining processes according to Pure-Tone Audiometry (PTA).

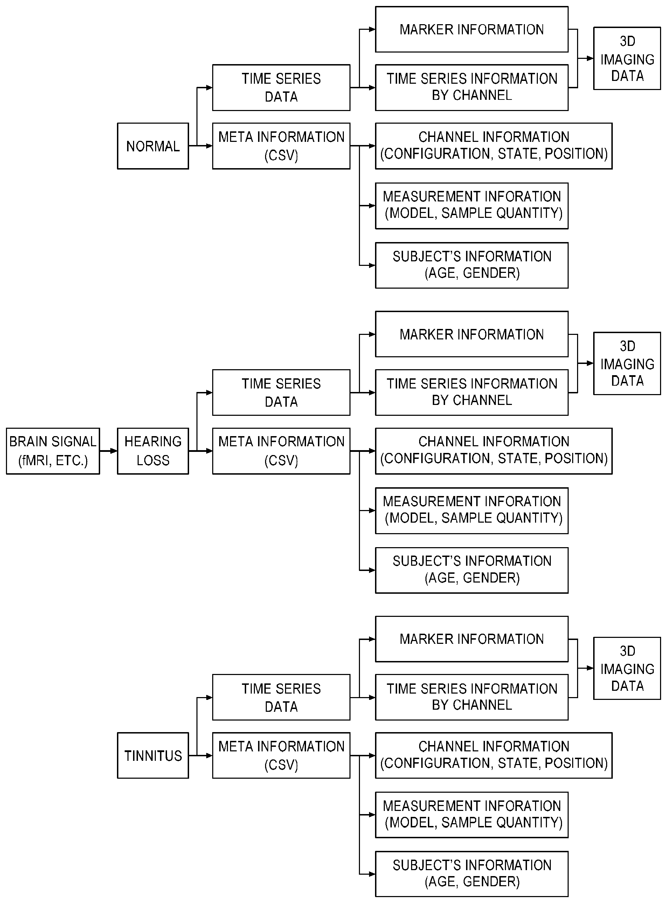

FIG. 23 shows one example adopting a structure of a brain signal activated by PTA.

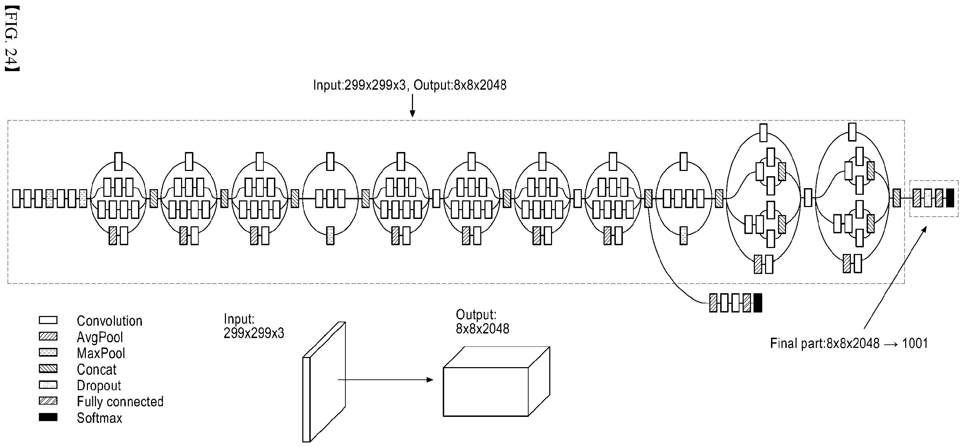

FIG. 24 shows one example of Active Brain Medical Biometric AI data model/algorithm.

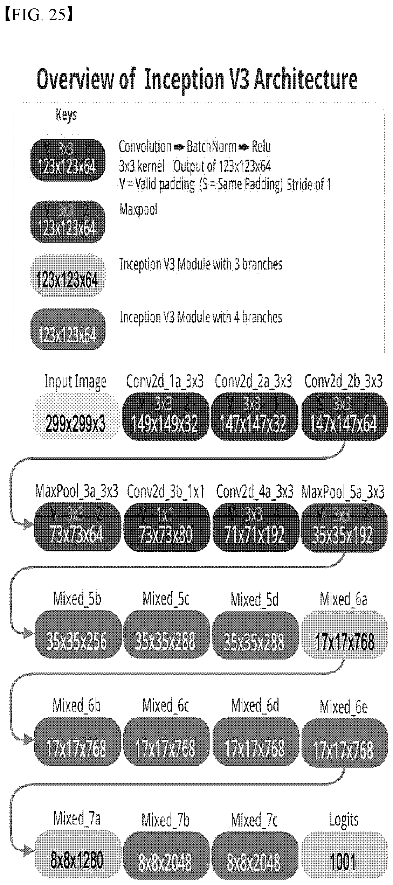

FIG. 25 shows one example of a structure of Inception V3.

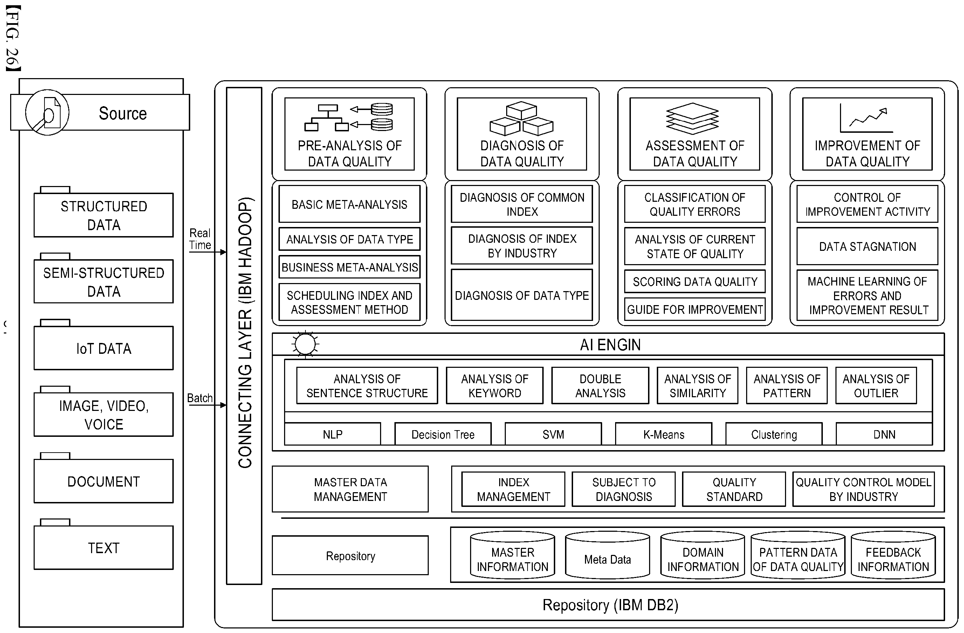

FIG. 26 show one embodiment of a concept map of SW of a way of the quality control and verification of AI learning data.

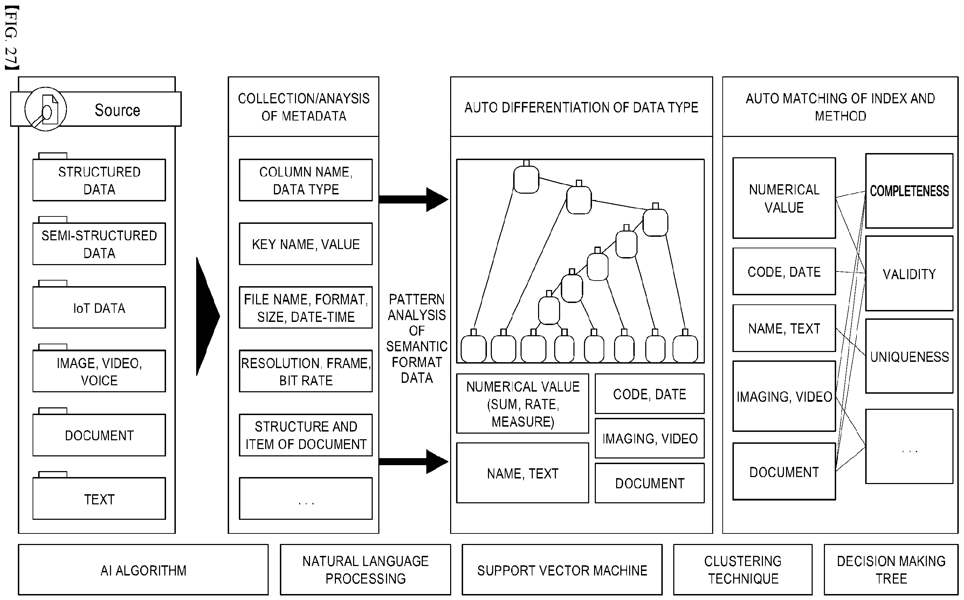

FIG. 27 show one embodiment of a pretreatment of the way of the quality control and verification of AI learning data.

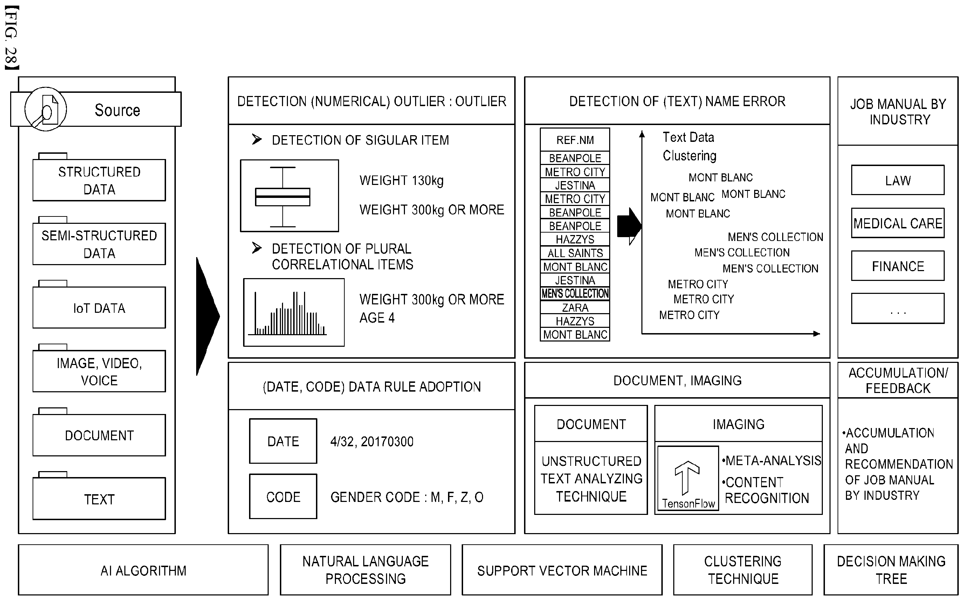

FIG. 28 shows one example of a diagnosis of the way of the quality control and verification of AI learning data.

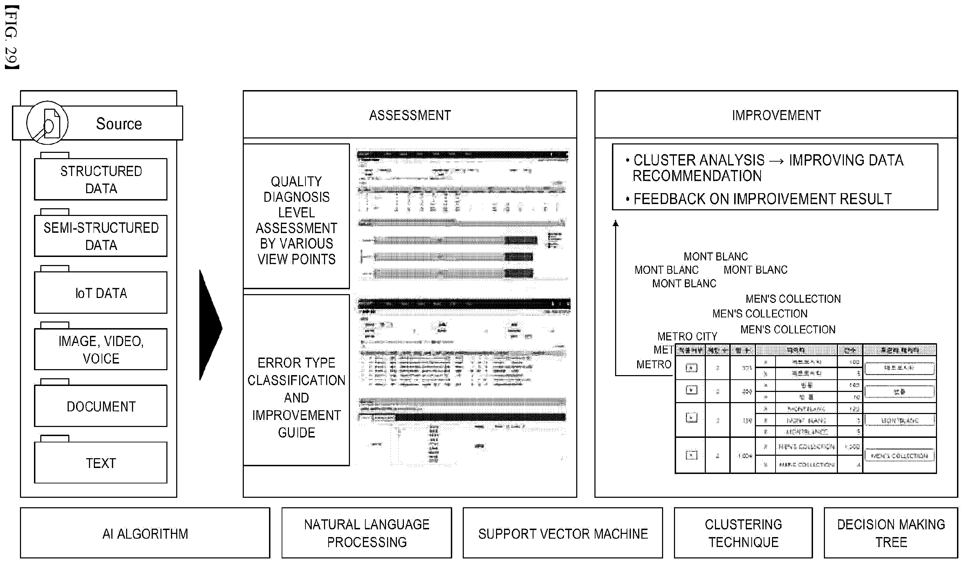

FIG. 29 shows one example of the way for controlling the quality of and verifying AI learning data.

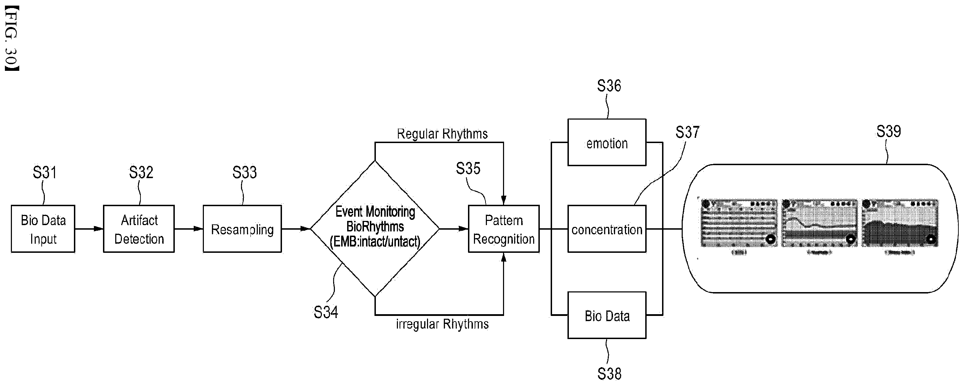

FIG. 30 shows one example of the detection and structurization of a factor for collecting bio data.

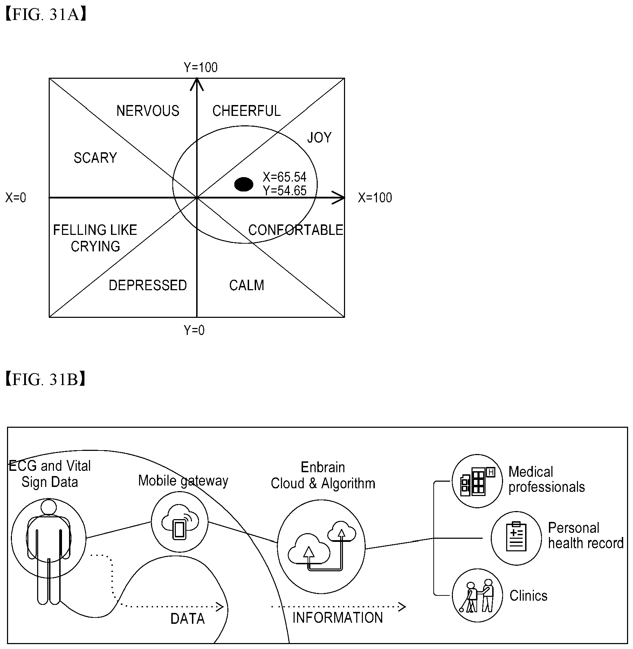

FIG. 31A and FIG. 31B show an example of a link plan of the collected data and one example of a design for emotion mapping.

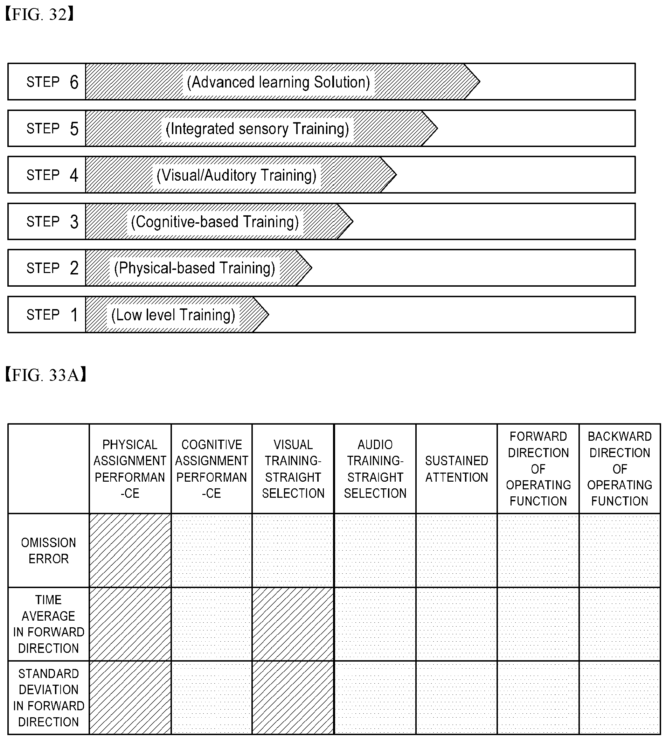

FIG. 32 shows one example of a training program divided into 6 steps according to a measurement result of child's brain development.

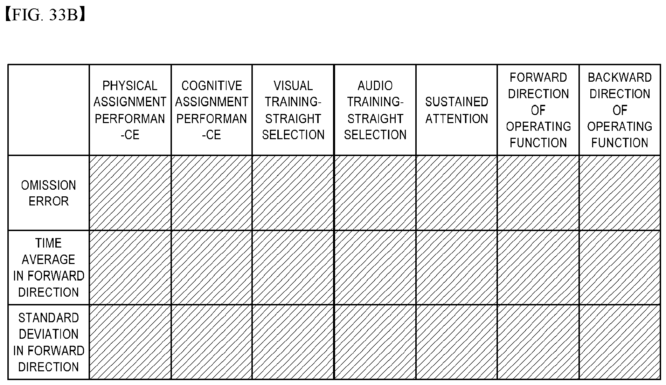

FIG. 33A and FIG. 33B show examples of respective program results before and after training.

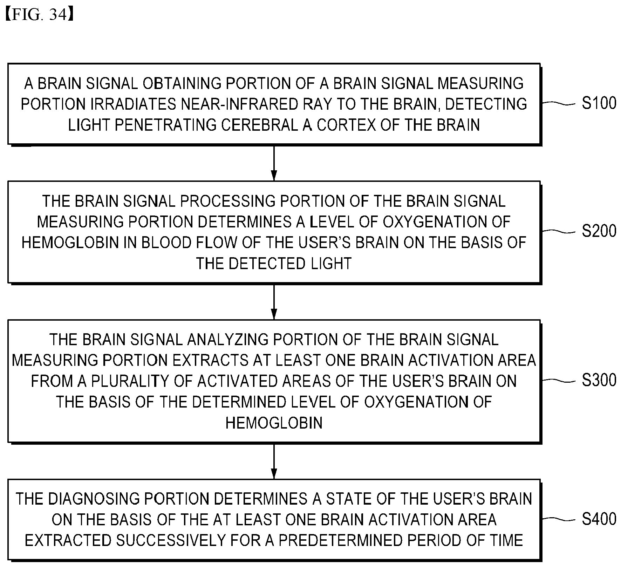

FIG. 34 is a flowchart explaining a method for collecting a brain related signal in a state that a user is moving and determining the user's brain state on the basis thereof, followed by providing feedback thereon.

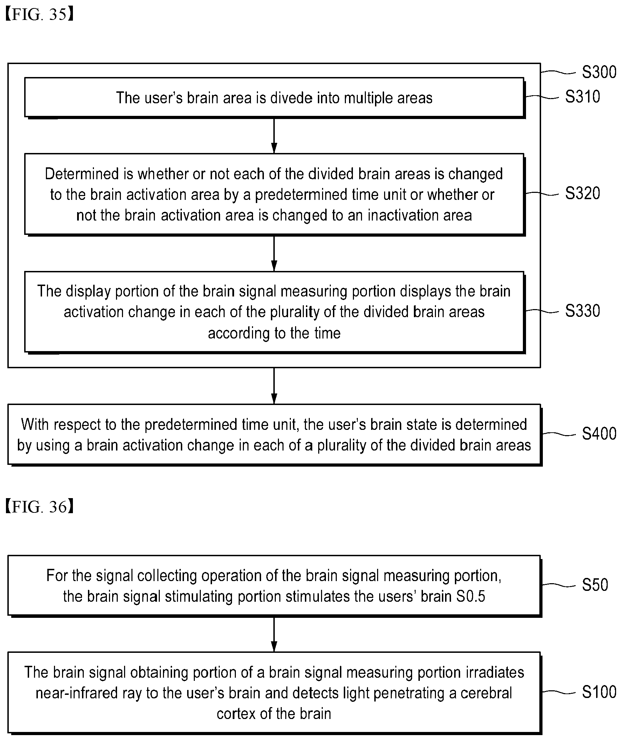

FIG. 35 is a flowchart explaining a method for displaying a brain activation change in each of a plurality of divided brain areas by time and determining the user's brain state therefrom.
FIG. 36 is a flowchart explaining a method for stimulating the user's brain for a signal collecting operation of the brain measuring portion.

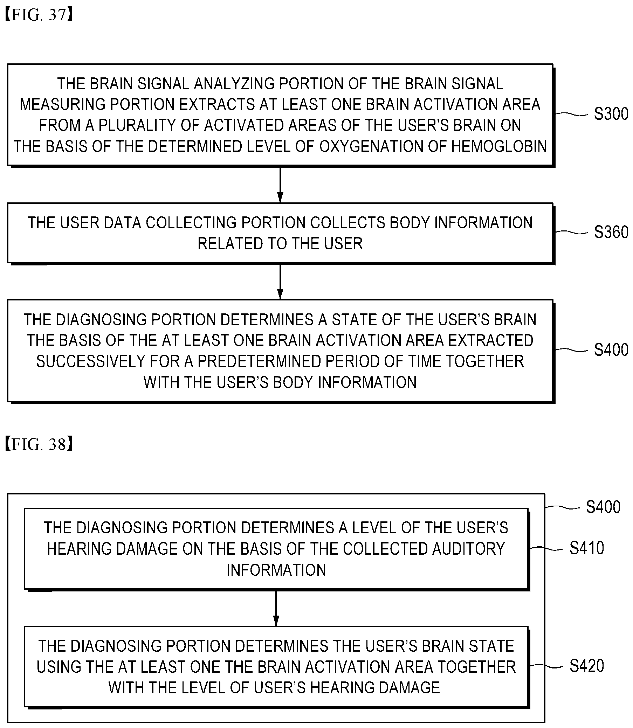

FIG. 37 is a flowchart explaining a method for determining the user's brain state by collecting body information related to the user and additionally using this information.
FIG. 38 is a flowchart explaining a method for determining the user's brain state by determining a level of hearing damage and additionally using this level.

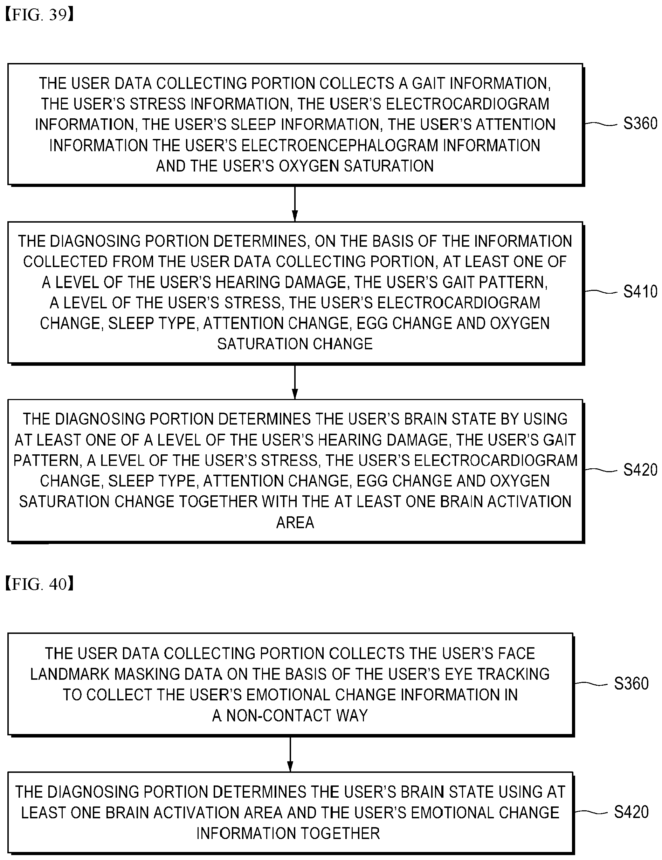

FIG. 39 is a flowchart explaining a method for determining the user's brain state by additionally using a gait information collecting portion that collects the user's gait information collects the user's gait information, a stress collecting portion that collect the user's stress information, an electrocardiogram information collecting portion that collects the user's electrocardiogram information, a sleep information collecting portion that collects the user's sleep information and a attention information collecting portion that collects the user's attention information.
FIG. 40 is a flowchart explaining a method for determining the user's brain state by additionally using a non-contact typed emotional change collecting portion on the basis of eye tracking.

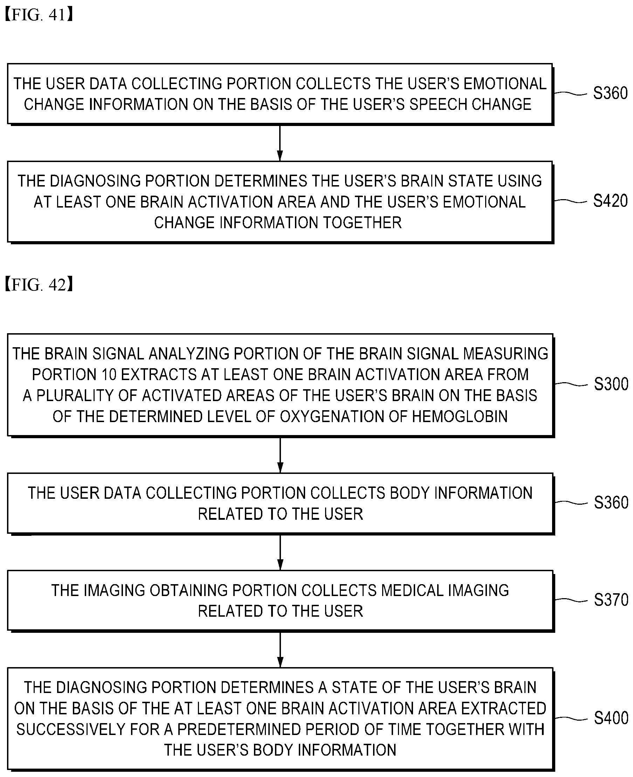

FIG. 41 is a flowchart explaining a method for determining the user's brain state by additionally using a non-contact typed emotional change collecting portion on the basis of speech change.
FIG. 42 is a flowchart explaining a method for determining the user's brain state by additionally collecting and utilizing medical imaging related to the user.

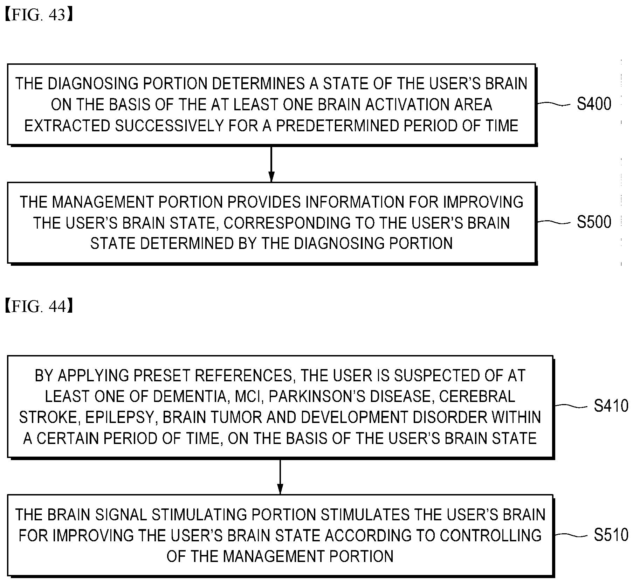

FIG. 43 is a flowchart explaining method for providing information for improving the user's brain state corresponding to the user's brain state.
FIG. 44 is a flowchart explaining a method for stimulating the brain in order to improve the user's brain state.

### DETAILED DESCRIPTION OF THE EMBODIMENTS

### Degenerative Brain Disease

The elderly population ratio (aged 65 or over) in Korea was 13.2% of the total population as of year 2019, however, as the aged population is increasing rapidly, it will be expected to reach 20% as of year 2025.

For the next 45 years, each proportion of a youth population and a working age population is expected to be decrease 3.7%p and 23.3%p, respectively and the elderly population ratio is expected to occupy 40.1% in the total population in 2060.

It is expected that elder care expenses in Korea will increase continuously, reaching 2 times of those in US by 2050 and furthermore this will the highest in the world along with Japan in 2060.

A spectacular increase in the population with dementia to be foreseen in the super-aged society appears and the prevalence rate of dementia shows an increasing trend with age.

Accordingly, dementia is one of urgent problems to be overcome in the modern world that will be entered into the super-aged society before long. The number of the world's dementia prevalence is 4,600 which is greater than the population in Spain, showing a two-fold increase every 20 years and thus will increase rapidly to 130,000,000 by 2050.

It is expected that the East Asian population with dementia will increase 193% by 2050 and the number of domestic patients with dementia is estimated to reach 2,710,000 by 2050.

Accordingly, national dementia care expenses increase resulting in causing social and economic burdens. The spectacular increase in patients with dementia causes an increase in national dementia care expenses. The national dementia care expenses approximated trillion won as of the year 2019 (about 0.7% of GDP) and will increase 2 times every 10 years. Thus, this is estimated to exceed 100 trillion won in 2050 (about 2.0% of GDP).

These economic expenses resulting from dementia are 2 times and 3 times of those for cardia disease and cancer, respectively. The worldwide social · economic expenses was 81.8 billion dollar as of the year 2015 and is expected to increase to 2 trillion dollars by 2030.

Domestic social · economic losses approximate 2 trillion won per year, increasing 2 times or more during recent 4 years (Reference: Search Report of Korean Institute for health & Welfare Policy).

The worldwide aging population increases the incidence of degenerative brain diseases and social problems. Even though many studies on the treatment and prevention of degenerative brain diseases including dementia are being conducted actively, these are at a standstill. Currently, dementia diagnosis is available through a thorough medical checkup in big hospitals and university hospitals, resulting in problems in terms of psychological, geographical and economic accessibility.

The current screening rate of dementia is 45% (for reference, prevalence rates of 4 major types of cancers are 90% or more), non-invasive dementia diagnosis methods are proposed in various fields. However, there has been not any study on a diagnosis method of dementia on the basis of information gathered through real-time monitoring.

Further, a failure rate of developing a treatment agent for dementia approximates 9.6%, and solanezumab expected as a treatment agent targeting amyloid beta has failed to show promise in Phase III trials.

The reason why a success rate of developing a treatment agent of dementia does not reach 1% is that no onset mechanism was found clearly.

### Problems of Prior Diagnosis Technique

The current dementia diagnosis is made up through comprehensive determination on the basis of various tests including brain imaging, electroencephalography, blood test, cerebral spinal fluid examination, Neuropsychological test, etc.

In regard of expenses, the brain imaging is widely used because brain's form such as cerebral atrophy can be seen directly. This uses various kinds of imaging techniques are used including Magnetic Resonance Imaging (MRI), Single-Photon Emission Computed Tomography (SPECT), Positron Emission Tomography (PET), etc., the PET imaging helping early diagnosis.

The electroencephalography checks overall cognitive decline by examining memory, attention, visuospatial sense, calculation ability, etc. This is used for early diagnosing a disease, monitoring disease progression and determining efficacies of treatment drugs.

However, the most major drawback of this examination is that the accuracy rate of the examination is proportional to the progression rate for dementia, this meaning that the rate of misdiagnosis is high at very early stage showing no clear symptom.

Further, in a case of cerebral spinal fluid examination, biomarkers accumulated in the brain may be detected directly. However, there is a drawback that it is very painful and inconvenience to collect cerebral spinal fluid for a subject.

### Problems of Prior Art related to Child's Brain Development

As of the year 2019, each population with intellectual disabilities, autism and ADHA is 213,000, 29,000 and 2,060,000, respectively.

The intellectual disabilities and the autism are slow of treatment effects, while in a case of the ADHA, appropriate treatments during childhood avoids continuing this to adulthood. Thus, early diagnosis and treatment of the ADHA is required.

In order to measure a child's brain state with existing MRI, fMRI, CT, etc., it is required that the child hardly moves, differently from his/her developmental features. In a case of toddlers, it is essentially required to administer a sleeping dug and an anesthetic drug.

At this time, when a baby aged less than 2 years is exposed to the anesthetic drug several times, a risk of the occurrence of permanent learning impairment increases 2 times. Further, in order to develop various kinds of new medicines for brain development, it is required to invest a lot of time and to collect biodata for a certain period of time.

There is a method to check the brain development through multilateral interaction but in a case of the existing product, hyperscanning for measuring a brain state multilaterally is not allowable and thus diversity of such a method for measuring the brain state is required. Accordingly, demands for developing products satisfying this requirement and mobile instruments for measuring the brain state are increasing.

### Objects of the Invention

Therefore, the present invention intends to contribute to solve social problems of neurologic diseases through building of a deep-learning dataset for early diagnosis and treatment of degenerative brain diseases increasing following aging population.

That is, in order to solve aforementioned problems, required are techniques for Active MRI brain activity essessement of a person with dementia and for early diagnosis of dementia.

Since clinical symptoms are observed long before occurrence of deteriorations in brain structure and function, it is important to essess or diagnose dementia early through the measurement of brain's ability to function.

In order for AI's recognition and understanding of its own accord, it is important to obtain a large scale of AI learning data processed in a form that an AI SW is capable of understanding association between things, AI performance based on machine learning is determined according to utilization of a huge amount of data collected under various circumstances and it is also important that there is synergy between the data and the AI.

According to another object of the present invention, provided are, for a child, "a multilateral wireless brain signal measurement device for measuring brain in real time" and a "brain development examination service".

In order to identify child's brain development state and to held child grow up healthy, the technique of the present invention is provided 1) to prevent and diagnose brain diseases such as ADHA, and 2) to provide experience value for cultivating child's attention in everyday life and learning.

Further, according to the present invention, provided are a device capable of collecting measure results of brain state for a certain period of time and a solution providing a brain developing program which is, on the basis of the collected data, capable of predicting brain disease risk and is adoptable to everyday life and learning.

According to another object of the present invention, provided is a sensitivity cognitive/sharing AI (Human-centered AI) service utilizing the construction of a multimodal emotional data network and the N-dimensional emotion mapping.

The emotion cognitive technique adopts a technique which defines an event stream (user's behavior) utilizing ICT and intelligence quotients for social emotional cognition and emotion sharing through a non-contact vital reaction and which is capable of recognize basic emotion and social emotion in a space of the N dimensional emotion mapping.

Further, the non-contact vital reaction is capable of utilizing measurement of a vital signal based five senses by using a sensor camera, an AI speaker, etc. which are usable in everyday life.

Further, the present invention provides and builds a data network which is capable of extending the user's behavior event and non-contact vital reaction step by step from a unimodal typed data network to the multimodal data network through which being capable of mapping with the social emotion and is also capable of growing up and evolving through AI.

Further, the emotion AI service provides a whole new level of human-centered AI service which overcomes unpredictable behavior or emotion of a learner or an emotional labor from a cognitive level to an intuitive level.

In order to achieve the aforementioned objects, the present invention intends to provide an AI based method for providing brain information which collects signals related with the brain of a user in a state of activity and determines the state of the user's brain therefrom and is capable of providing feedback on the brain information.

Further, the present invention intends to provide a system and method for providing brain information, the system comprising: a brain signal measuring portion that includes a brain signal obtaining portion which irradiates near-infrared ray to user's brain and detects light penetrating the cerebral cortex of the brain, a brain signal processing portion which determines a level of oxygenation of hemoglobin in blood flow of the user's brain on the basis of the detected light and a brain signal analyzing portion which extract at least one brain activation area from a plurality of areas of the user's brain on the basis of the determined level of oxygenation of hemoglobin; and a diagnosing portion that determines the state of the user's brain on the basis of the at least one brain activation area which was extracted successively for a predetermined period of time.

Further, the present invention intends to provide the system and method for providing brain information, the system including a brain signal stimulating portion for stimulating the user's brain.

Further, the present invention intends to provide the system and method for providing brain information, the system determining the state of the user's brain more precisely by additionally using an auditory information collecting portion that collects the user's auditory information, a gait information collecting portion that collects the user's gait information, an electrocardiogram information collecting portion that collects the user's electrocardiogram information, a sleep information collecting portion that collects the user's sleep information, a attention information collecting portion that collects the user's attention information, a non-contact typed emotional change information collecting portion that collects the user's face landmark masking data on the basis of the user's eye tracking to collect the user's emotional change information in a non-contact way, a non-contact typed emotional change information collecting portion that collects the user's emotional change information on the basis of the user's speech change, etc.

Further, the present invention intends to provide the system and method for providing brain information, the system determining the state of the user's brain more precisely by additionally using an imaging collecting portion that collects medical imaging related to the user and thus using the brain activation area, body information related to the user and the collected medical imaging related to the user together.

Further, the present invention intends to provide the system and method for providing brain information, the system comprising a diagnosing portion that is capable of determining brain diseases including dementia, Parkinson's disease, cerebral stroke, Epilepsy, brain tumor and development disorder, and a management portion that provides information for improving brain state, corresponding to the user's brain state determined by the diagnosing portion.

Further, the present invention intends to provide the system and method for providing brain information, the system further using a brain signal stimulating portion to stimulate the user's brain when the user, , by applying preset references, is suspected of at least one of dementia, Parkinson's disease, cerebral stroke, Epilepsy, brain tumor and development disorder within a certain period of time, on the basis of the user's brain state.

Hereinafter, the AI based device and method for providing brain information according to preferable embodiments of the present invention will be described in detail with the accompanying drawings.

Hereinafter, preferable embodiments of the present invention will be described with the accompanying drawings. Further, the content of claimed inventions are not limited to the preferable embodiments to be described and whole configuration to be explained in the present embodiments are not essential as a solution.

### Artificial Intelligence(AI) based Device for Providing Brain Information

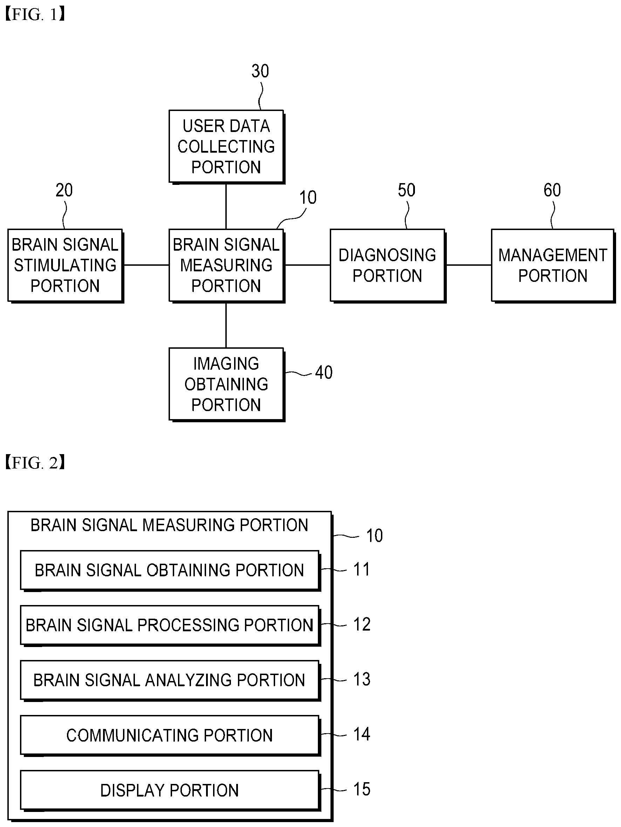

FIG. 1 is a block diagram showing an Artificial Intelligence (AI) based device for providing brain information.

Referring to FIG. 1, the AI based device 1 for providing brain information according to the present invention may include a brain signal measuring portion 10, a brain signal stimulating portion 20, a user data collecting portion 30, an imaging obtaining portion 40, a diagnosing portion 50 and a management portion 60.

Firstly, the brain signal measuring portion 10 collects signals related to users' brain.

In particular, the brain signal measuring portion 10 according to the present invention irradiates near-infrared ray to the user's brain and detects light penetrating the cerebral cortex of the brain, determines a level of oxygenation of hemoglobin in blood flow of the user's brain on the basis of the detected light and extracts at least one brain activation area from a plurality of areas of the user's brain on the basis of the determined level of oxygenation of hemoglobin.

The present invention is characterized in that a signal collecting operation of the brain signal measuring portion 10 may be performed in a state that the user is moving.

Moving on to the next, the brain signal stimulating portion 20 stimulates the user's brain for the signal collecting operation of the brain signal measuring portion 10.

Furthermore, the brain signal stimulating portion 20 stimulates the user's brain for improving the user's brain state according to controlling of the management portion.

Further, the user data collecting portion 30 collects body information related to the user.

The body information related to the user may include the user's auditory information and gait information, a level of the user's stress, and the user's electrocardiogram information, sleep type, attention change, etc.

Further, the imaging obtaining portion 40 collects medical imaging related to the user.

The imaging obtaining portion 40 according to the present invention may use an MRI imaging scan obtaining portion that collects an MRI imaging scan related to the user; a CT imaging scan obtaining portion that collects a CT imaging scan related to the user; and an fMRI imaging scan obtaining portion that collects an fMRI imaging scan related to the user.

Further, the diagnosing portion 50 determines the user's brain state on the basis of the collected signals from the brain signal measuring portion 10.

The diagnosing portion 50 may determine the user's brain diseases including dementia, Parkinson's disease, cerebral stroke, Epilepsy, brain tumor and development disorder.

Lastly, the management portion 60 provides information for improving the user's brain state, corresponding to the user's brain state determined by the diagnosing portion 50.

For example, when the user, by applying preset references, is suspected of at least one of dementia, Parkinson's disease, cerebral stroke, Epilepsy, brain tumor and development disorder within a certain period of time, on the basis of the user's brain state, a user's brain stimulating operation of the brain signal stimulating portion 20 may be triggered for improving the user's brain state according to controlling of the management portion 60.

Hereinafter, respective configurational elements will be described in regard of specific structures and functions with reference to drawings.

### Brain signal measuring portion

FIG. 2 is a block diagram of a brain signal measuring portion that is a configurational element of the AI based device for providing brain information.

Referring to FIG. 2, the brain signal measuring portion 10 may include a brain signal obtaining portion 11, a brain signal processing portion 12, a brain signal analyzing portion 12, a communicating portion 14 and a display portion 15.

Firstly, the brain signal obtaining portion 11 irradiates near-infrared ray to the user's brain and detects light penetrating the cerebral cortex of the brain.

Wherein, a signal collecting operation of the brain signal obtaining portion 11 is performed in a state that the user is moving.

Moving on to the next, the brain signal processing portion 12 determines a level of oxygenation of hemoglobin in blood flow of the user's brain on the basis of the detected light.

The brain signal processing portion 12 extracts oxygenated hemoglobin (Oxy Hb) concentration and deoxygenated hemoglobin (Deoxy Hb) concentration in blood flow of the user to determine a level of oxygenation of hemoglobin.

Further, the brain signal analyzing portion 13 extract at least one brain activation area from a plurality of areas of the user's brain on the basis of the determined level of oxygenation of hemoglobin.

Wherein, in the at least one brain activation area, oxygen transferred by the Oxy Hb in blood flow is consumed to decrease the Oxy Hb concentration and to increase the Deoxy Hb concentration corresponding to the decreased Oxy Hb concentration.

The Oxy Hb concentration and the Deoxy Hb concentration have an optical property that changes in a visible-ray area and a near-infrared area and consequentially, the brain signal measuring portion 10 collects the signal through functional Near-infrared Spectroscopy (fNIRS).

Meanwhile, the brain signal analyzing portion 13 divides the user's brain area into multiple areas and determines whether or not each of the divided brain areas is changed to the brain activation area by a predetermined time unit or whether or not the brain activation area is changed to an inactivation area.

Based thereon, the diagnosing portion 50 may determine the state of the user's brain using brain activation changes of the respective divided brain areas with reference to a predetermined period of time.

Further, the communicating portion 14 builds networks between the brain signal measuring portion 10 and other configurational elements (the brain signal stimulating portion 20, the user data collecting portion 30, the imaging obtaining portion 40, the diagnosing portion 50, the management portion 60, etc.) to support data communication.

Usable wireless communication technique may include Wireless LAN (WLAN) (Wi-Fi), Wireless broadband (Wibro), World Interoperability for Microwave Access (Wimax), High Speed Downlink Packet Access (HSDPA).

Further, usable short range communication technique may include Bluetooth, Radio Frequency Identification (RFID), Infrared Dara Association (IrDA), Ultra-Widenband (UWB), ZigBee, etc.

Further, the display portion 15 displays the brain activation change in each of the plurality of the divided brain areas according to the time.

The display portion 15 may include at least one of a Liquid Crystal Display (LCD), a Thin Film Transistor-Liquid Crystal Display (TFT LCD), an Organic Light-Emitting Diode (OLED), a flexible display and a 3D display.

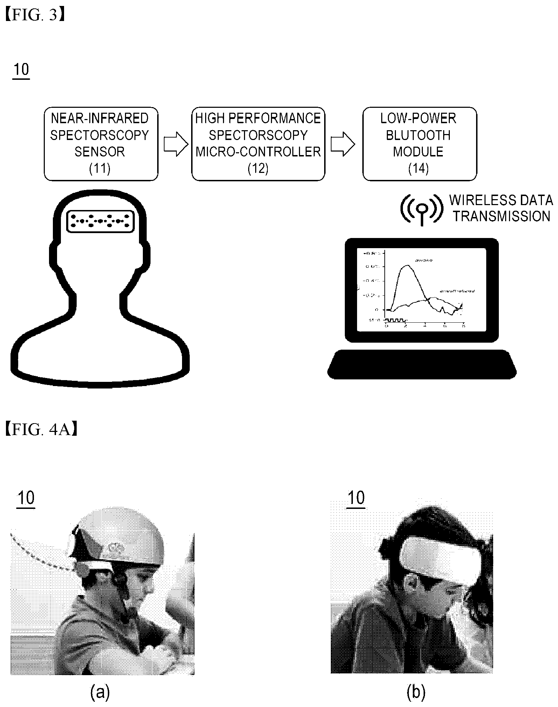

FIG. 3 is a view for explaining the operation of the brain signal measuring portion.

Referring to FIG. 3, the user's brain state and development state thereof are measured multilaterally in real time in a state that the user is moving. That is, a brain signal measuring device is provided which is capable of measuring brain signals of a multiuser so as to analyze the brain signal wirelessly in real time without any limitation.

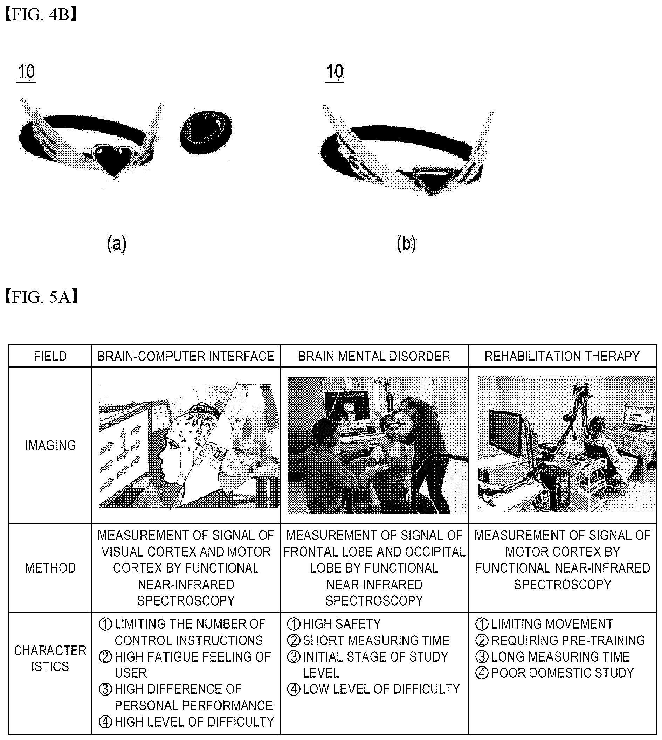

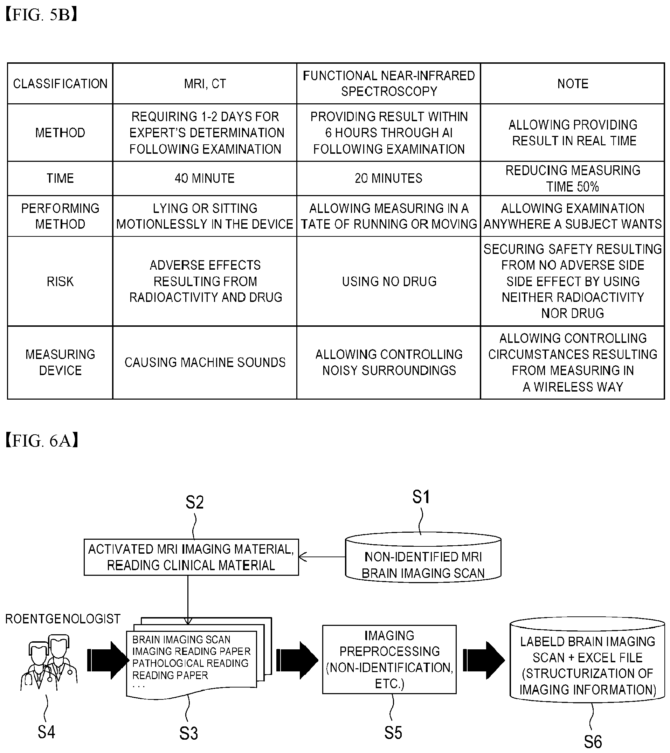

Meanwhile, each of FIG. 4A and 5B shows one example of a type of the brain signal measuring portion.

The brain signal measuring portion 10 according to the present invention may be a full cover type which covers the whole of the user's head as shown in (a) of FIG. 4A, alternatively a front type which is adhered closely to a front part of the user's head as shown in (b) of FIG. 4A.

As considering usability of the brain signal measuring portion, particularly being used for a child, custom UI/UX custom designs for providing experience value may be considered by adopting a child custom design to the brain signal measuring portion 10 and making up user friendly environment.

In the present invention, a wearable technology for measuring brain signals may be applied which uses bluetooth low energy (BLE) allowing being used for a long time. Further, a flexible substrate is used for designing the brain signal measuring device for a child to fit baby's head shape, allowing weight minimization of the device.

Further, utilized are a brain signal analyzing algorithm that is capable of measuring attention and brain development of the user in terms of physiology through brain activation analyzation, a data transmission algorithm that is capable of securing data reliability even if a channel state is changed, and a brain activation area cognitive algorithm.

Meanwhile, each of FIG. 5A and FIG. 5B shows a view for explaining functional near-infrared spectroscopy to be adopted to the present invention.

Referring to FIG. 5A, in the functional Near-infrared spectroscopy (fNIRS) technique applied to the present invention, fNIRS is an imaging technique which irradiates light of the near-infrared area having a wave length of 650 to 1000nm and detects light penetrating a brain tissue of the brain to measure a change of the hemoglobin concentration according to a change non-invasively in blood flow of the brain without any risk resulting from a surgery.

The fNIRS technique applied to the present invention uses a functional fNIRS for performing real-time brain measurement under the living circumstance in which a patient is able to moving rather than being fixed.

The fNIRS is an imaging technique which irradiates light of the near-infrared area having a wave length of 650 to 1000nm and detects light penetrating a brain tissue of the brain to measure a change of the hemoglobin concentration according to a change non-invasively in blood flow of the brain without any risk resulting from a surgery.

This functional fNIRS may shows a result in real time differently from the existing assessment methods, reduce costs for buying a device and maintenance fee thereof, and increase reliability of a measurement result as the measurement result shows a sociality directly.

FIG. 5B show a comparison between the current brain measuring technique and the fNIRS applied to the present invention.

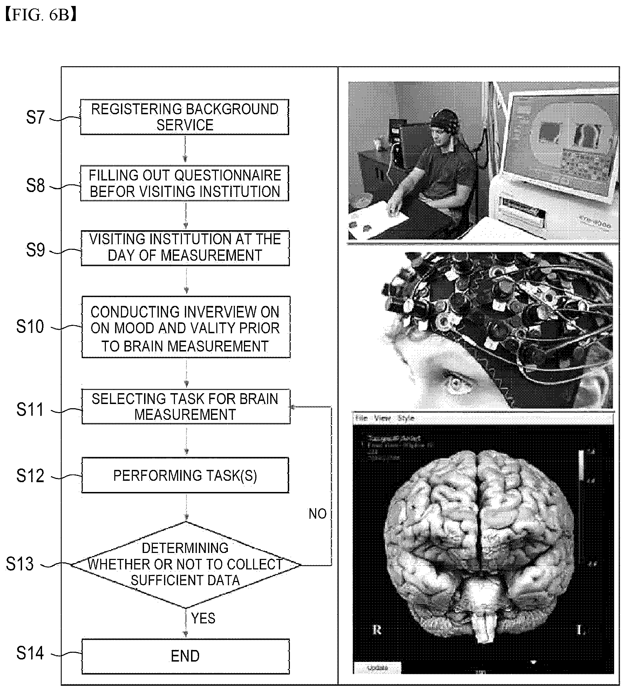

Meanwhile, each of FIG. 6A and FIG. 6B is a flowchart for explaining a measuring operation of the brain signal measuring portion.

Referring to FIG. 6A, a non-identified MRI brain imaging scan is collected (S1), imaging data of activated MRI brain scan technique and reviewed clinical data is collected (S2) and feedback is provided by a radiologist (S4) followed by putting them together (S3), and the clinical information may be structuralized into labeled brain imaging scan and a file in excel format (S6) through imaging pretreatment (S5).

That is, through collecting the imaging scan of the inactivated brain MRI for MRI diagnosis and the clinical information from the medical database operated by a hospital, references for hearing loss, tinnitus and furthermore dementia may be defined by utilizing imaging scan reading and pathological reading.

Further, a diagnosis result of the MRI imaging scan is divided into data of a normal PSAP result and an imaging scan of brain MRI. Database construction may be performed under a specific circumstance because there may be an error in the reading even when diagnosing hearing loss, tinnitus and normalcy.

As a particular reference for hearing loss to be described below, a brain imaging scan of a patient diagnosed as hearing loss by the PSAP examination may be used. As a particular reference for tinnitus, a brain imaging scan of a patient diagnosed as tinnitus by the PASAP examination may be used. As a particular reference of normalcy, a brain image scan of a patient diagnosed as normalcy by the PSAP examination may be used.

Further, FIG. 6B shows one example of a protocol including data collecting and measuring processes.

Referring to FIG. 6B, a background service is registered (S7), a questionnaire is filled out before visiting an institution (S9), an interview on mood and vitality is conducted prior to brain measurement (S10), a task for the brain measurement is selected (S11) followed by performing the selected task (S12), determination on whether or not to collect sufficient data is made (S13) and the brain measurement is finished (S14) or the step S11 is restarted according to determination.

Further, the imaging data to be applied to the present invention may be defined as imaging data of activated brain MRI scan.

Further, non-identification of imaging data may be processed by a non-identifying method and a non-identifying tag structure (RAW data).

Further, the meta data to be applied to the present invention collects patient's meta information for the imaging information and is utilized for quality inspection through additional information in case of patients with hearing loss and tinnitus to secure reliability.

Meanwhile, FIG. 7 shows one example of a whole system of the brain signal measuring portion.

In FIG. 7, (a) shows one example of a data collecting device, (b) shows one example that a user wears a fNIRS device, and (c) shows one example of time series data of the signal of the fNIRS device.

Referring to (a) to (c) of FIG.7, it is found that the brain activation area is checked safely without any surgery by irradiating near infrared ray to the brain, followed by detecting light penetrating cerebral cortex and processing the detected light to measure attentions of Oxy Hb and Deoxy Hb.

Further, the system neither has riskiness in using nor is affected by ambient noise.

Further, the system has high convenience and portability and is cost-effective compared to other brain function imaging devices.

FIG. 8 shows one example of a signal measured by the brain signal measuring portion.

In FIG. 8, shown is each block average of time series by channel position in the fNIRS.

FIG. 9 shows one example visualizing and representing the signal measured by the brain signal measuring portion as brain related information.

In FIG. 9, shown is one example that the block averages of the time series of the signal in the fNIRS device by channel position are mapped on the head.

In the present invention, in order for being applied to machine learning, a data configuration for preventing algorithm bias and a method for collecting data may be used as follow.

Sample bias may occur when the collected data represents an environment in which the AI system is expected to be performed or this does not appear.

Generally, algorithm learns a data set of a subset deliberately selected from a whole data set rather than learning the whole thereof. Thus, in order to reduce the sample bias, it is important to select a subset that is capable of representing the whole of the data set besides a sufficiently large dump data set when selecting the data set of the subset.

Further, measurement bias occurs when the distortion of value is caused systematically by a device used in observing or measuring something, this kind of bias likely to distort data in a certain way.

An instrument with measurement bias is not capable of duplicating an environment in which a model is operated, this causing that the learning data distorts actual data resulting in a biased result. The measurement bias is not avoidable even if collecting more data.

Accordingly, in order to solve the aforementioned problems, the present invention is allowable to configure an environment not resulting in biased results to collect the data when collecting data.

Meanwhile, each FIG. 10A to FIG. 10C shows one example of the signal measured by the brain signal measuring portion, visualized information and processed information.

FIG. 10A shows an inactivated MRI brain imaging scan in which it is allowable to create imaging of a vital tissue having a different distribution rate of water and fat by measuring the distribution of hydrogen atoms through MRI based on that distribution rates of water and fat are different according to human tissues (bones, muscles, intestines, etc.).

However, through such information like FIG. 10A, it is not allowable to figure out a phenomenon when a person behaves or is in a state of moving.

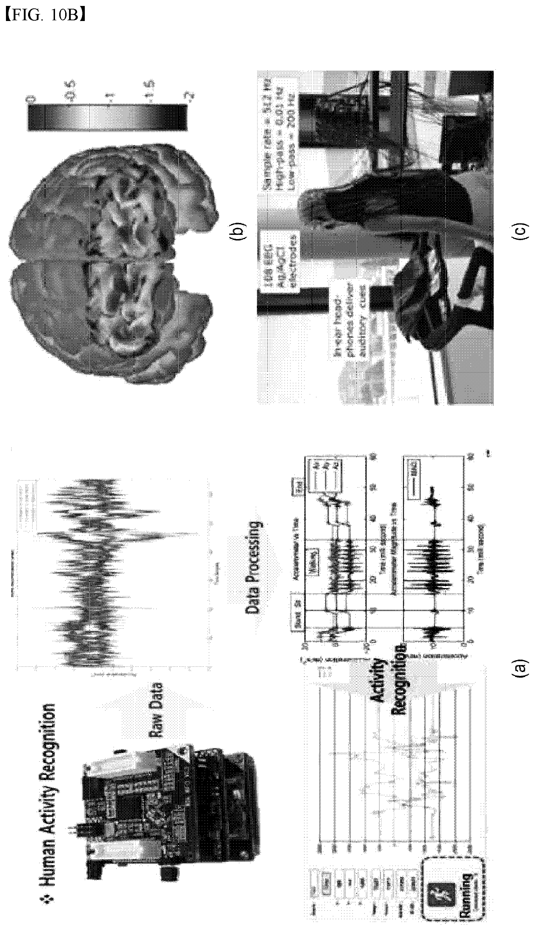

On the contrary, FIG. 10B shows one example of an activated MRI brain imaging scan according to the present invention.

In FIG. 10B, the block averages of the time series of the signal in the fNIRS device by channel position are mapped on the head.

A non-invasive method for measuring brain function, fNIRS measures a change in blood flow of the brain non-invasively by using near infrared ray, the measurement principle being based on measurement of a level of oxygenation of hemoglobin in blood flow using near infrared ray.

As irradiating near infrared ray to a living body and detecting a fNIRS signal penetrating the tissue, followed by processing, it is allowable to measure an Oxy Hb concentration and a Deoxy Hb concentration.

An activation area of the brain consumes oxygen transferred by the Oxy Hb in the blood flow and Oxy Hb is changed into Deoxy Hb. Two such hemoglobins have an optical property that changes in a visible-ray area and a near-infrared area, and attention thereof measured by fNIRS is usable as a criterion for brain activity.

Such a method according to the present invention is capable of measuring brain function in a state that a person is running or moving and of providing a result with a short period of time after examination.

Further, the method is capable of measuring without any medicine and of controlling ambient sound.

FIG. 10C shows one example of a result for cleaning the time series data of the signal of fNIRS.

In FIG. 10C, for building an appropriate model, variables having the highest explanation ability is selected from variables which are determined to have high correlation with an item subjected to abnormality detection.

Further, adjust R^2 for each subset is measured to select an item made up of the least number of variables from the subsets which have the largest value.

Meanwhile, each of FIG. 11A to FIG. 11C shows one example of an authoring tool for the operation management of AI learning data.

The authoring too is required in order to build AI learning data effectively.

That is, a labeling function is required which automatically tags and labels labelling project information, imaging subjected to labelling, a labelling list, a reference imaging, etc., stored in the server with an imaging labelling application solution.

As a way of an authoring tool for the operation management of the AI learning data, imaging adding, storing and creating functions may be used.

As shown in FIG. 11A, imaging desired to be examined may be added using a relevant authoring tool. Following adding brain or fracture MRI imaging scan using a portable MRI device, additional imaging storing and creating functions may be grafted by pressing a plus button.

Further, an authoring tool utilizing an annotation function is capable of performing a tagging operation for the imaging additionally stored and created and an additional information recording operation through a note function for i.e. peculiarity, etc.

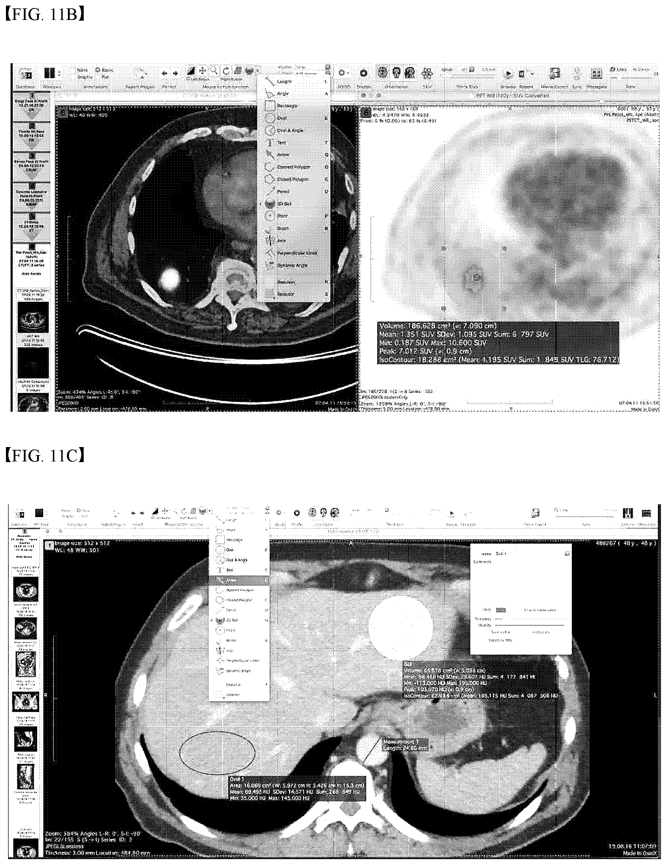

FIG. 11B and FIG. 11C show one example of a case of utilization of the annotation function.

As a way of utilization for the operation management of the AI learning data, a cross validation may be performed to verify an exemplary reading model of the activated MRI brain imaging scan for diagnosing normalcy, hearing loss and tinnitus.

Further, a performance is assessed by using various reading criteria the criteria including measurement of sensitivity, specificity and an Area Under a Receiver operating Characteristic Curve (AUROC), thereby capable of building the exemplary reading model.

As aforementioned, the brain signal measuring portion 10 collects information related to the user's brain and the brain signal collecting operation of the brain signal measuring portion 10 is performed in a state that the user is moving.

Meanwhile, the diagnosing portion 50 determines a state of the user's brain on the basis of the collected signal.

In particular, the diagnosing portion 50 may determine the state of the user's brain on the basis of at least one brain activation area which was extracted successively for a predetermined period of time.

Herein, the diagnosing portion 50 is capable of diagnosing the user's brain disease, the brain disease including dementia, Parkinson's disease, cerebral stroke, Epilepsy, brain tumor and development disorder.

Furthermore, the management portion 60 may be utilized additionally which provides information for improving the user's brain state, corresponding to the user's brain state determined by the diagnosing portion 50.

For example, when the user, by applying preset references, is suspected of at least one of dementia, Parkinson's disease, cerebral stroke, Epilepsy, brain tumor and development disorder within a certain period of time, on the basis of the user's brain state, the management portion 60 controls the brain signal stimulating portion 20 to be described below to provide a signal for stimulating the user's brain so as to improve the user's brain state.

### Brain signal stimulating portion

The brain signal stimulating portion 20 provides a function of stimulating the user's brain for the signal collecting operation of the brain signal measuring portion 10.

Furthermore, the brain signal stimulating portion 20 may provide a function of stimulating the user's brain according to controlling of the management portion 10 for improving the user's brain state.

FIG. 12 is a block diagram of a brain signal stimulating portion, that is, a configurational element of the AI based device for providing brain information. FIG. 13 is a block diagram of a brain activating device of the brain signal stimulating portion.

The brain signal stimulating portion 20 intensively stimulates a synapse meaning a part in which brain cells are connected to communicate with each other. In order to activate a brain area as long-term memory, the brain area should be stimulated through a place, emotion and a story.

Accordingly, the brain signal stimulating portion 20 reinforces activity of the brain area targeted intensively through multidimensional environmental stimulations on the basis of a natural object disposed in a space wherein activities, such as exhibition, music, motion, etc., are made and selectively activates a function of the brain area. According to classification criteria of Diagnostic and Statistical manual of mental Disorders (DSM-V) defined by American Psychiatric Association, the brain area may be classified into attention, visuospatial ability, memory, executive ability, verbal ability, calculation ability and sound cognitive ability, etc.

That is, the present invention intensively reinforces various activities of the brain area, such as attention, empathy, creativity, memory, healing, etc. through the natural object, particularly a plant and multi-dimensional environmental stimulation such as sound, sense of touch, scent, sense of sight, memory, etc. and activates functions.

As shown in FIG. 12 and FIG. 13, a brain activation system 10 according to a preferable embodiment of the present invention includes a brain activating device 24 and a management server 21 in order to intensively reinforce an activity of a pre-targeted brain area within a predetermined space where the natural object was installed and to activate a function of the brain area, the brain activating device applying systemized multi-dimensional environmental stimulation and the management server controlling driving of the brain activating device 24 to apply the multi-dimensional environmental stimulation to the targeted brain area in the space.

The brain activating device 24 may include a media portion 30, an acoustic portion 40, a lighting portion 50 and a scent transferring portion 60 in order to intensively reinforce the activity of the pre-targeted brain area within the predetermined space where the natural object was installed and to activate the function of the brain area, the media portion implementing media art corresponding to a concept desired to be created in the space, the acoustic portion outputting sound, the lighting portion irradiating light and the scent transferring portion transferring a scent to the user.

FIG. 14 FIG. 14 shows one example building the brain signal stimulating portion spatially.

In the embodiment according to FIG. 14, a space, for example, an exhibition space 11 implements media art which makes the user give attention to intrinsic nature, that is, inner world along with light illuminated inside the exhibition space 11, takes imagined forest as a main theme to concentrate on a new one's own inner world found inside the space i.e. ego and stimulates stories and senses through a plurality of spaces having concepts different with each other.

As the above, the exhibition space 11 may be created through landscape installation art and real flower arrangement, projection mapping, light art, interactive contents and sculptures (objets).

For example, as shown in FIG. 14, the exhibition space 11 may be divided into five spaces including first to fourth spaces 12 to 15 and a fifth space 16 implementing an experience space and a photo tunnel, the five spaces having their own concept.

The first to fifth spaces 12 to 16 stimulate sympathetic nerve through various colors of lights, objets and media art so as to increase psychological satisfaction for that the user desires to explore the user's own ego.

For this, the brain activating device 24 may make the user be absorbed in the space where various sculptures and media objets resulting from the movement of intense colors and drive the user to commune with the natural object into which dreamlike media melt.

For example, the concept of the first space 12 may be 'INTRO - Awaken Your brain', the concept of the second space 13 may be 'STATE - Turning Back Your Nature', the concept of the third space 14 may be 'Creative - Fee Your Nature', the concept of the fourth space may be 'Healing - That's Me!' and the concept of the fifth space may be 'Active - Vivid & Rejuvenation' .

That is, the first space 12 stimulates synapse intensively to activate the brain, the second space 13 provides unfamiliar stimulation to check the state of the brain, the third space 14 stimulate the brain through scent in order for pruning, the fourth space 15, as a space which the user faces in third-person while passing through from the first space 12 to the third space 14, offers healing to the brain, the fifth space 16 rejuvenates senses of the brain through experience.

The media portion 30 may include first to fifth media modules 31 to 35 which implement media art corresponding to the concept of each of the first and fifth spaces 12 to 16, and the acoustic portion 40 may include first to fifth acoustic modules 41 to 45 which output sound corresponding to the concept of each of the first and fifth spaces 12 to 16.

Similarly, the lighting portion 50 may include first to fifth lighting modules 51 to 55 which irradiate light and the scent transferring portion 60 may include first to fifth transferring modules 61 to 65 which transfer scent of the natural object such as a tree and a flower, disposed inside the exhibition space 11.

At this time, the respective media modules 31 to 35 may be a device that displays media art such as photo or a video in various ways, for example, a projector which projects the media art to the respective spaces, the surface of a wall and the natural object or a display panel.

The respective acoustic modules 41 to 45 may include a speaker and an amplifier which output sound including natural white noise.

The respective lighting modules 51 to 55 may be composed of a combination of one or two of a laser generator, an LED and a neon lighting.

The respective transferring modules 61 to 65 may an air blower which blows air to transfer the scent generated from the natural object disposed in each of the spaces or a copy scent thereof to the user of each of the spaces.

Wherein, at least one or more of the respective modules may be provided in each of the divided spaces.

Meanwhile, FIG. 15 shows one example of a flowchart explaining a brain activating method of the brain signal stimulating portion.

In the step S10 of FIG. 15, the brain activating device 24 transfers media art, sound, lighting and scent corresponding to the concept of each of the divided spaces of the exhibition space 11 i.e. the first to fifth spaces 12 to 16.

When the user enters the first space 12 of the exhibition space 11, the respective first media module, acoustic module, lighting module and transferring module 31 to 61 provided in the first space 12 output media art, sound and lighting corresponding to the concept of the first space 12 and the natural object such as a plant and an objet, disposed in the first space 12 and transfer scent to the user.

Accordingly, the brain activating device 24 stimulates synapse through the user's multi-dimensional senses of sight, hearing, touch, smell, etc. (S12).

In the step S14, when the user enters the second space 13, the respective second media module, acoustic module, lighting module and transferring module 32 to 62 provided in the second space 13 output media art, sound and lighting corresponding to the concept of the second space 13 and the natural object such as a plant and an objet, disposed in the second space 13 and transfer scent to the user.

Accordingly, the brain activating device 24 implements neural network driving the user to commune with the exhibition space 11.

In the step S16, when the user enters the third space 14, the respective third media module, acoustic module, lighting module and transferring module 33 to 63 provided in the third space 14 output media art, sound and lighting corresponding to the concept of the third space 14 and the natural object such as a plant and an objet, disposed in the third space 14 and transfer scent to the user.

Accordingly, the brain activating device 24 guides a moving line to the user and prunes the user's brain function.

The respective fourth media module, acoustic module, lighting module and transferring module 34 to 64 provided in the fourth space 15 output media art, sound and lighting corresponding to the concept of the fourth space 15 and the natural object such as a plant and an objet, disposed in the fourth space 15 and transfer scent to the user.

Accordingly, the brain activating device 24 offers healing to the user through relaxation of the brain while walking on a path through the forest created vividly.

In the Step S20, when the user enter the fifth space 16, the respective fifth media module, acoustic module, lighting module and transferring module 35 to 65 provided in the fifth space 16 output media art, sound and lighting corresponding to the concept of the fifth space 16 and the natural object such as a plant and an objet, disposed in the fifth space 16 and transfer scent to the user.

Accordingly, the brain activating device 24 stimulate the user's five senses of sighting, touch, ect., through the natural object created with various nature-based finishing materials and a media art fairy tale including the user's, particularly child's direct touch experience and a story, and provides a photo tunnel.

Meanwhile, in the embodiment according to the present invention, in order to describe configurations of the first to fifth spaces, the respective spaces are described successively, however, a participation order of the user is not limited thereto.

That is, the user entered the second space via the first space may be move to the fourth space or the fifth space as well as the third space along following an arrow shown in FIG. 3.

Further, the user may move from the third space to the fifth space as well as to the fourth space, alternatively moving to the second space.

Further, the user may move from the fourth space to the fifth space, also moving to the second space or the third space again.

Further, the user may move from the fifth space to the second space, the third space or the fourth space again.

Accordingly, it is allowable to move the respective spaces optionally rather than according to a predetermined order. As moving to the participated space again resulting in repeated participation, the brain area activated in the respective spaces may be activated intensively and the function thereof may be reinforced. As the above processes, the present invention is capable of activating brain function of a required part through the natural object disposed in the exhibition space and the multi-dimensional stimulation.

Further, the present invention is capable of activating various brain functions, such as attention, empathy, creativity, memory, healing, etc. through the natural object, particularly a plant and multi-dimensional environmental stimulation such as sound, sense of touch, scent, sense of sight, memory, etc.

### User data collecting portion, Imaging obtaining portion, Diagnosing portion and Management portion

The user data collecting portion 30 collects body information related to a user.

The body information related to the user may include the user's auditory information and gait information, a level of the user's stress, and the user's electrocardiogram information, sleep type, attention change, etc.

FIG. 16A shows a block diagram of a user data collecting portion.

Referring to FIG. 16A, the user data collecting portion 30 may include an auditory information collection portion 31 that collects the user's auditory information.

At this time, the diagnosing portion 50 determines a level of hearing damage of the user on the basis of the collected auditory information and may determine the user's brain state using at least one brain activation area and the determined level of hearing damage of the user.

Particularly, when the user's hearing damage results from hearing loss, the diagnosing portion 50 may determine the user's brain state by additionally using the body information related to the user, an average value of pure-tone thresholds in both of the user's ears, a level of the user's hearing loss and information related to the auditory information collecting portion.

As another example, when the user's hearing damage results from Tinnitus, the diagnosing portion 50 may determine the user's brain state by additionally using the body information related to the user, an average value of pure-tone thresholds in both of the user's ears, a level of the user's hearing loss and information related to the auditory information collecting portion.

Meanwhile, the brain signal collecting operation of the brain signal measuring portion 10 may be performed in a state that the user's hearing damage occurs while the user is moving.

Further, the user data collecting portion 30 may include a gait information collecting portion 32 that collects the user's gait information collects the user's gait information, a stress collecting portion 33 that collect the user's stress information, an electrocardiogram information collecting portion 34 that collects the user's electrocardiogram information, a sleep information collecting portion 35 that collects the user's sleep information and a attention information collecting portion 36 that collects the user's attention information.

Further, the user data collecting portion 30 may further include a brainwave information collecting portion that collects the user's electroencephalogram (EGG) information and an oxygen saturation information collecting portion that collects the user's oxygen saturation (not illustrated).

At this time, the diagnosing portion 50 may determine, on the basis of the collected auditory information, at least one of a level of the user's hearing damage, the user's gait pattern, a level of the user's stress, the user's electrocardiogram change, sleep type, attention change, EGG change and oxygen saturation change.

Further, the diagnosing portion 50 may determine the user's brain state using the at least one of the level of the user's hearing damage, the user's gait pattern, the level of the user's stress, the user's electrocardiogram change, sleep type, attention change, EGG change and oxygen saturation change together with the at least one brain activation area.

Further, the signal collecting operation of the brain signal measuring portion 10 may be performed under the circumstance being applied with at least one of states that the user's gait information is changing, the user's stress is changing, the user's electrocardiogram is changing, the user's sleep condition is changing and the user's attention is changing, while the user is moving.

Further, the user data collecting portion 30 may include a non-contact typed emotional change information collecting portion 37.

This non-contact typed emotional change information collecting portion 37 may collect the user's face landmark masking data on the basis of the user's eye tracking to collect the user's emotional change information in a non-contact way.

At this time, the diagnosing portion 50 may determine the user's brain state using the at least one brain activation area together with the user's emotional change information.

Further, non-contact typed emotional change information collecting portion 37 may collect the user's emotional change information on the basis of the user's speech change.

At this time, the diagnosing portion 50 may determine the user's brain state using the at least one brain activation area together with the user's emotional change information.

Particularly, the diagnosing portion 50 may determine the user's brain state on the basis of the at least one brain activation area extracted successively for a predetermined period of time.

The diagnosing portion 50 may determine the user's brain disease including dementia, Parkinson's disease, cerebral stroke, Epilepsy, brain tumor and development disorder.

Furthermore, the diagnosing portion 50 may additionally utilize the management portion 60 that provides information for improving the user's brain state, corresponding to the user's brain state determined by the diagnosing portion.

Hereinafter, as a representative of the user data collecting portion 30, the auditory information collecting portion 31 and the non-contact typed emotional change collecting portion 37 will be described.

Firstly, an embodiment using the auditory information collecting portion 31 is described in detail.

FIG. 17A to FIG. 17D are views explaining correlation between hearing loss and dementia and FIG. 18 is a view explaining brain volume loss resulting from Alzheimer's disease.

Further, FIG. 19 is a view explaining correlation between cognitive function damage resulting from hearing impairment and dementia.

Referring to FIG. 17A to FIG. 17D, as the aged society results in a spectacular increase in the population with dementia and Korea enters the aged society, dementia becomes an issue in the youth population besides the aged population.

Particularly, the proportion of patients with severe dementia to overall dementia patients shows a reduction trend, i.e. 32% as of the year 2016 and 30% as of the year 2018, through medication treatment and various programs for severe dementia patients. This means that symptoms of the severe dementia patients are improved whereas early stage of dementia shows a growing trend.

Noise is an unwanted sound considered unpleasant and loud to hearing. Sensorineural hearing loss caused by this noise is referred to as 'noise-induce hearing loss".

The root cause lies on damage of the sensory organ, that is, cochlea. Particularly, this often occurs as a consequence of damaged outer hair cells. A sound of 75dB or lower hardly incudes hearing loss, however, a sound pressure of environment noise in the office or conversation circumstance approximates 60dB, a noise level inside the cabin of buses and subway and restaurants approximating 80dB, the maximum volume of the earphones of MP3s or portable CD players approximating 100dB, a noise level of airplanes approximating 140dB and a noise level of gunfire approximating 170dB. When listening through earphones at a volume to the extent that the next person can hear, the volume level ranges approximately from 100dB to 115dB. Prolonged exposure to a noise of 85dB or higher may cause damages on ears.

Exposures to a sound pressure of 100dB for 15 minutes or longer without any protection equipment and to a sound pressure of 110dB regularly for 1 minute or longer cause risk of hearing loss. In the light of that ordinary noise level inside the cabin of buses and subway is 80dB, the youth should maintain music sound to 90db or higher in order to hear music, the hearing loss occurring as a consequence of repeated exposures thereto.

Sound intensity is determined by the amplitude of a sound wave and the scale for measuring intensity is decibel (dB) scale. For every 10dB increase in the sound intensity, a noise level is increased two times. Usually, a sound intensity of 75dB or lower does not affect hearing damage.

As symptoms of noise-induced hearing loss, when a person has noise-induced hearing loss, he/she is likely to turn on a television, a radio, etc, at a high volume and is often referred to as 'Saojeong' in Korean meaning a person who is slow in understanding what someone says or is partially deaf.

Further, the person with noise-induced hearing loss hardly understands what the other person says accurately even in a little noisy surrounding. Commonly, he/she shows decrease in hearing at 4kHz and is more likely to be accompanied by tinnitus.

Further, in addition to hearing damage and tinnitus, the person with noise-induced hearing loss may feel unpleasant and anxiety, suffers from insomnia, headache and is stressed, this disrupting his/her normal life. Even worse, the noise-induced hearing loss affects pulsation and blood pressure to cause maldigestion and autonomic dysfunction.

The noise-induced hearing loss is diagnosed by performing pure-tone threshold test and an audiometry which measures measuring hearing sensitivity accurately by a frequency, the audiometry including tinnitus test, otoacoustic emission test, loudness recruitment test, auditory brainstem response test, etc. Following prolonged exposure to noises, hearing loss, particularly to the sound of a high frequency of 4kHz appears gradually.

The damaged hearing due to the induced-hearing loss is basically irreducible because damaged auditory cells are not repaired. Particularly, if difficulty hearing in an ear occurs suddenly within a few days, this may be sudden sensorineural hearing loss and thus the person should be subjected to more precise examinations as well as treatments with steroid hormones, vasodilators, antiviral agents, etc.

In such a case, it is necessarily required to relieve the ear for a certain period of time for the recovery thereof.

The sudden hearing loss is referred to as sensorineural hearing loss that occurs suddenly without any clear causes, this occurring in one ear commonly but also appearing in both ears extraordinarily. Occasionally, this is accompanied by tinnitus and dizziness.

Generally, the sudden hearing loss is regarded as an urgent disease and needs to start to admission treatment. Supposed causes of the onset may include viral infection occurring in the auditory nerve, blood flow disorder in the inner ear, disruption or damage to the cochlea, immune disorders of the inner ear, neuropathological disease, tumor, ototoxic drugs, etc.

Acoustic tumor may occupy 1 to 2% of patients with the sudden hearing loss. This is diagnosed through MRI and a curative rate thereof is high in a patient who was subjected to the treatment at an early stage through combined modality therapy of adrenal cortical steroids, vasodilators, blood flow improving agents, metabolism improving agents, sedatives, etc. as well as dietary therapy such as low salt diet and high protein diet, while taking a relief in a hospital.

As a result of this treatment, a third of the patients is completed recovered, while hearing power is improved but not recovered completely to normal in another a third and no treatment effect may show in the rest.

Referring to FIG. 18 and FIG. 19, risks of dementia are two times and three times higher in each of the elderly with mild hearing damage and middle level of hearing damage, respectively.

In a person with severe hearing damage, a risk of dementia is five times higher than persons without hearing damage. According to Lancet report, hearing loss occupies 3% of causes of the onset of dementia, ranked first among 9 potential risk factors thereof.

The hearing loss may be an early symptom of Alzheimer disease showing decline verbal ability in a state that there are background noises.

Further, the hearing loss increases a demand for cognitive resources and the brain has to work harder when auditory ability deteriorated.

Understanding and tries to understand conversation demand that the brain 'borrows' cognitive ability from other parts thereof, particularly memory. Basically, as receiving more information, the information transferred to the memory becomes decreased.

The hearing loss causes brain tissue remodeling and/or relative deprivation resulting in decline cognitive ability and the decline auditory ability often results in impaired social interaction and social engagement disorder.

Accordingly, there was a study result that the progression of dementia may be slowed 75% in a fashion of environmental approach and through preventing decreases in cognitive ability required for interaction and hearing loss.

Further, it is very essential to detect and treat hearing loss early for preventing and reducing decline cognitive ability.

Risks of dementia in each of the elderly with mild hearing loss and severe hearing loss appear two times and five times higher, respectively as compared to the population with normal hearing ability (Lin, Albert, 2014).

FIG. 20 is a view explaining correlation between hearing loss (tinnitus) and Alzheimer's disease (dementia).

Referring to FIG. 20, 30% of the population with hearing loss experienced tinnitus, requiring the treatment of hearing loss together with the examination and treatment of tinnitus (Healthy hearing, 2019). As a result of examination over patients with tinnitus, the tinnitus affected auditory functions first, followed by affecting cognitive function (particularly attention and thinking ability) and dementia, sequentially.

In conclusion, it is likely that the tinnitus leads to dementia (Mahoney, Rohrer, Goll, Fox, Rossor, Warren, 2010).

As a result of analysis of 18 literatures from 1996 to 2014, the tinnitus decreases cognitive intelligence while increasing anxiety and depression (Tegg-Quinn, Bennett, Elikelboom, & Baguley, 2016).

FIG. 21 is a view explaining correlation between hearing and cognitive impairment.

Referring to the steps S21 to S24 of FIG. 21, predisposition to cognitive impairment increases two times every 30dB increase when the auditory damage occurs.

Particularly, each difference of 30dB and 60dB increases predisposition to dementia two times and 4 times, respectively (Uhlmann, Rees, 1989), and the cognitive function is declined according to every change of 25dB (Result of follow-up examination over 347 persons for 5 years, Lin, Ferrucci, Metter, An, Zonderman, & Resnick, 2011).

In conclusion, this leads to problems with hearing > sentence hearing > word hearing > overall comprehension > responses > mild cognitive impairment (MCI) in order.

When there is auditory damage at every 30dB, a predisposition to cognitive impairment approximates 2 times, this occurring when a problem arises in at least on of hearing, cognition and sight rather than sequentially (LEE, Su-jeong, LEE, Seung-jin, SONG, Ji-yeon, KIM, Hyeong-hee, 2014).

FIG. 22 is a view explaining processes according to Pure-Tone Audiometry (PTA). FIG. 23 shows one example adopting a structure of a brain signal activated by PTA. Referring to FIG. 23, the user data collecting 30 may include the auditory information collecting portion 31 31.

At this time, the diagnosing portion 50 may determine a level of the user's hearing damage on the basis of the collected auditory information and may determine the user's brain state using the at least one the brain activation area together with the level of user's hearing damage.

Particularly, when the user's hearing damage results from hearing loss, the diagnosing portion may determine the user's brain state by additionally using the body information related to the user, an average value of pure-tone thresholds in both of the user's ears, a level of the user's hearing loss and information related to the auditory information collecting portion.

As another example, when the user's hearing damage results from Tinnitus, the diagnosing portion may determine the user's brain state by additionally using the body information related to the user, an average value of pure-tone thresholds in both of the user's ears, a level of the user's hearing loss and information related to the auditory information collecting portion.

Meanwhile, a signal collecting operation of the brain signal measuring portion 10 may be performed in a state that the user's hearing is damaged, while the user is moving.

Moving on to the next, a function of the diagnosing portion 50 using the non-contact typed emotional change collection portion 37 will be described.

Bio data for personal emotion recognition may be collected and the data argumentation may be made based on the non-contact typed emotional change collecting portion 37.

Texts and facial expression data may be collected which show 6 types of basic feelings for various domains (age, gender, existence of accessories) in a uniform distribution.

A large scale of daily speechpose data and labelling data showing those emotions may be collected in the light of gender, age, region, for the 6 types of basic emotions (anger, disgust, fear, joy, sadness, surprise) and emotional neutrality.

A basic emotion label may be tagged on the collected data and collecting face landmark masking data including eye tracking.

Further, pose estimation imaging may be collected for understanding an object's intension and a speech argumentation method may be provided for correcting the emotion data imbalance.

Regarding bio data based personal emotion recognition, emotions may be recognized through a trained Deep Neural Network (DNN) model using the collected data.

In order to increase an emotion recognition performance, it is allowable to develop and apply preprocessing methods such as noise removal, speech part extraction, etc. and modules for selecting and extracting speech characteristics suitable for the emotion recognition.

Further, an utterance level emotion recognition model based on deep learning is applicable and extraction of time series of speech is allowable to infer a basic emotion for the object data and to recognize personal emotion utilizing the speech data.

Among deep learning methodologies, an emotion recognition AI model is applicable which utilizes recurrent neural network taking account of time series, a transformer using attention mechanism, etc. Optimal emotional recognition is allowable by using a designed emotion recognition AI model in machine learning and hyperparameter tuning method such as Bayesian optimization.

Further, it is allowable to recognize contours of eyes, a nose, lips, eyebrows and face, and also to extract attention from the collected eye tracking data and face landmark time series (it is allowable to use attention based artificial neural network due to using of consecutive data).

It is allowable to apply a social emotion recognition method through tracking personal emotion and daily situation and to advance emotion embedding and represent motions in consecutive spaces rather than classification thereof. Also, it is allowable to apply a multimodal emotion analysis by reinforcing an embedding space reflecting continuity of the time series data and combining emotion embeddings extracted from sight, speech and natural language.

For social emotion recognition, personal emotion extraction and vectorization techniques are applicable by suing texts from speech, d-vector based speaker embedding, existing speech emotion recognition techniques.

It is allowable to apply characteristics extracted by Cepstral mean and variance normalization (CMVN) for suppressing performance decline for the speech of daily conversation with uneven tones resulting from emotions and also to apply a language model applying domain adoption capable of taking account of characteristics of the expression and word which are changeable according to situation information.

The non-contact typed emotional change collecting portion 37 may use a non-contact typed emotional change collecting portion that collects the user's face landmark masking data on the basis of the user's eye tracking to collect the user's emotional change information in a non-contact way.

Wherein, the diagnosing portion 50 may determine the user's brain state using the at least one brain activation area together with the user's emotional change information.

Further, the non-contact typed emotional change collecting portion 37 may use a non-contact typed emotional change collecting portion that collects the user's emotional change information on the basis of the user's speech change.

Wherein, the diagnosing portion 50 may determine the user's brain state using the at least one brain activation area together with the user's emotional change information.

Particularly, the diagnosing portion 50 may determine a state of the user's brain on the basis of the at least one brain activation area extracted successively for a predetermined period of time.

Wherein, the diagnosing portion 50 may determine the user's brain disease, the brain disease including dementia, mild cognitive impairment (MCI), Parkinson's disease, depression, cerebral stroke, Epilepsy, brain tumor and development disorder.

Furthermore, a management portion 60 may be further utilized which provides information for improving the user's brain state, corresponding to the user's brain state determined by the diagnosing portion 50.

Meanwhile, FIG. 16b shows a block diagram of an imaging obtaining portion.

The imaging obtaining portion 40 according to the present invention may use an MRI imaging scan obtaining portion 41 that collects an MRI imaging scan related to the user, a CT imaging scan obtaining portion 42 that collects a CT imaging scan related to the user and an fMRI imaging scan obtaining portion 43 that collects an fMRI imaging scan related to the user.

Wherein, the diagnosing portion 50 may determine the user's brain state on the basis of the at least one brain activation area, body information related to the user and the collected medical imaging related to the user together.

The diagnosing portion 50 may determine the user's brain diseases including dementia, Parkinson's disease, cerebral stroke, Epilepsy, brain tumor and development disorder.

Lastly, the management portion 60 provides information for improving the user's brain state, corresponding to the user's brain state determined by the diagnosing portion 50.

For example, when the user, by applying preset references, is suspected of at least one of dementia, Parkinson's disease, cerebral stroke, Epilepsy, brain tumor and development disorder within a certain period of time, on the basis of the user's brain state, a user's brain stimulating operation of the brain signal stimulating portion 20 may be triggered for improving the user's brain state according to controlling of the management portion 60.

### AI utilizing application service

Active Brain Medical Biometric AI data shows high performance for analyzing various kinds of imaging and is capable of being applied with an AI reading exemplary model by using inception v3 among Convolutional neural network (CNN) based models which are widely used as imaging analyzing deep learning.

Since the present invention aims to provide basic data that is prominent for developing medical AI reading technology and to build a virtuous cycle of ecosystem that expands and opens data spontaneously according to advancements of AI technology in private and public corporations, the active Brain Medical Biometric AI data is required for verifying validation of the constructed data.

FIG. 24 shows one example of Active Brain Medical Biometric AI data model/algorithm.

Referring to FIG. 24, the Inception v3 is an imaging recognition model widely used, accomplishes 78.1% or higher accuracy in ImageNet dada set and needs supervised learning process using a large scale of label designated imaging aggregation.

The imaging includes a bounding box that designates more accurate position of an object designated with a label.

Further, FIG. 25 shows one example of a structure of Inception V3.

Referring to FIG. 25, learning and assessment data sets are divided intentionally and then maintained. Imaging of the learning data set is only used for model learning, while imaging of the estimation data set is only used for model accuracy assessment.

The model is made of symmetrical/asymmetrical configurational elements including convolution, average pooling, maximum pooling, joining, dropout, complete connection layers.

FIG. 26 show one embodiment of a concept map of SW of a way of the quality control and verification of AI learning data.

Referring to FIG. 26, pre-analysis, diagnosis, assessment and improvement of data quality is performed through real-time data connection with a source code. Data storing is performed by fitting a repository that is a storing device to reference information and quality control is performed by using the stored data in an AI engine.

FIG. 27 show one embodiment of a pretreatment of the way of the quality control and verification of AI learning data.

Referring to FIG. 27, automation is performed by quality control pre-analysis to minimize human cost. A data type dependent automatic classifying system is constructed allowing creating automatic matching of a landmark and a method.

FIG. 28 shows one example of a diagnosis of the way of the quality control and verification of AI learning data.

Referring to FIG. 28, various types of data diagnosing techniques are applicable by measuring data quality and data quality measurement is allowable according to texts, imaging, numerical numbers, waveforms, etc. Also, data accumulation and feedback provision are allowable according to industry dependent business rules (law, medical care, finance, etc.).

FIG. 29 shows one example of the way for controlling the quality of and verifying AI learning data.

Referring to FIG. 29, data quality assessment for lifecycle is allowed to progress by proceeding improvement and feedback. The assessment guides various viewpoint dependent quality assessments and the classification and improvement of error types. The improvement performs a recommendation of improvement data and feedback on an improvement result through cluster analysis.

It is allowable to identify learner's daily data and characteristics of a learning pattern with a data collecting method.

For example, it is allowable to figure out the data and bio data collectable from the learner's daily life, to divided an age group into total 10 grade groups from an age group of 6 years (preschool children) to an age group of 15, to detect and structuralize factors for collecting bio data, and to detect a prominent factor (behavior and speech) for collecting related data.

The steps S31 to S39 of FIG. 30 show one example of the detection and structurization of a factor for collecting bio data.

Further, it is allowable to apply determinants and classification to a design for an emotion mapping, these necessarily involved in clustering and emotion mapping for social emotion through the learner's characteristic dependent classification.

FIG. 31A shows an example for an accuracy of the emotion mapping.

Further, FIG. 31B show an example of a link plan of the collected data in which a collection of the bio data advanced to increase an accuracy may be involved for advancing reliable data transmission and network between data, and a collecting method may be adopted for increasing an accuracy of the data linked to social emotion and learning activity may be also adopted.

Meanwhile, FIG. 32 shows one example of a training program divided into 6 steps according to a measurement result of child's brain development.

This model is, for a child, "a multilateral wireless brain signal measurement device for measuring brain in real time" and a "brain development examination service".

Referring to FIG. 32, the present invention provides a personalized brain development program on the basis of a brain measurement result from the "multilateral wireless brain signal measurement device for measuring brain in real time" and the "brain development examination service".

That is, it is allowable to provide an application for providing a measurement result of a brain development state and a brain development training program may be provided, the brain development training program including, as a main content, a brain area (prefrontal activation level, occipital activation level, temporal activation level) required to be leveled up through attention measurement, and attention level derivation (Level I - 5 min., Level II - 10 min, Level III - 20min.).

Wherein, according to each level, it is allowable to provide appropriate attention training program (anxiety decline, realization of joy of immersion, distracting time decline) and brain changing program (physical assignment performance, cognitive assignment performance, visual training - straight selection, audio training - straight selection, sustained attention, forward direction/backward direction of operating function).

FIG. 33a and FIG. 33b show examples of respective program results before and after training.

In FIG. 33A, the color red considered to be below average indicates a problem in the attention, the color green considered to be over average indicating high attention. In FIG. 33b, the color green considered to be over average indicates that there is not any problem in the attention.

Accordingly, the diagnosing portion 50 may determine, on the basis of AI, a state of the user's brain by using brain activation area, body information related to the user and medical imaging related to the user. Subjected brain diseases include dementia, Parkinson's disease, cerebral stroke, Epilepsy, brain tumor and development disorder.

Further, the management portion 60 may provide information for improving the user's brain state, corresponding to the user's brain state determined by the diagnosing portion.

### Method for providing feedback on determined brain state

FIG. 34 is a flowchart explaining a method for collecting a brain related signal in a state that a user is moving and determining the user's brain state on the basis thereof, followed by providing feedback thereon.

Referring to FIG. 34, first of all, the brain signal obtaining portion 11 of the brain signal measuring portion 10 irradiates near-infrared ray to the brain, detecting light penetrating a cerebral cortex of the brain S100.

Particularly, in the step S100, a signal collecting operation of the brain signal obtaining portion 11 is performed in a state that the user is moving.

Wherein, as aforementioned, the brain signal measuring portion 10 may be implemented as a full cover type which covers the whole of the user's head, alternatively a front type which is adhered closely to a front part of the user's head.

Then, the brain signal processing portion 12 of the brain signal measuring portion 10 determines a level of oxygenation of hemoglobin in blood flow of the user's brain on the basis of the detected light S200.

Particularly, in the step S200, on the basis of the detected light, a level of oxygenation of hemoglobin is determined by extracting oxygenated hemoglobin (Oxy Hb) concentration and deoxygenated hemoglobin (Deoxy Hb) concentration in blood flow of the user.

Further, in at least one brain activation area, oxygen transferred by the Oxy Hb in the blood flow is consumed to decrease the Oxy Hb concentration and to increase the Deoxy Hb concentration corresponding to the decreased Oxy Hb concentration.

Thus, the Oxy Hb concentration and the Deoxy Hb concentration have an optical property that changes in a visible-ray area and a near-infrared area and the brain signal measuring portion according to the present invention collects the signal through functional Near-infrared Spectroscopy (fNIRS).

Following the step S200, the brain signal analyzing portion 13 of the brain signal measuring portion 10 extracts at least one brain activation area from a plurality of activated areas of the user's brain on the basis of the determined level of oxygenation of hemoglobin S300.

Then, the diagnosing portion 50 determines a state of the user's brain on the basis of the at least one brain activation area extracted successively for a predetermined period of time S400.

### Method for displaying and determining brain state

Hereinafter, particularly described is the step S300 in which the brain signal analyzing portion 13 of the brain signal measuring portion 10 extracts at least one brain activation area from a plurality of activated areas of the user's brain on the basis of the determined level of oxygenation of hemoglobin.

FIG. 35 is a flowchart explaining a method for displaying a brain activation change in each of a plurality of divided brain areas by time and determining the user's brain state therefrom.

Referring to FIG. 35, in the step S100, the user's brain area is divided into multiple areas S310, followed by determining whether or not each of the divided brain areas is changed to the brain activation area by a predetermined time unit or whether or not the brain activation area is changed to an inactivation area S320.

Then, the display portion 15 of the brain signal measuring portion 10 displays the brain activation change in each of the plurality of the divided brain areas according to the time S330.

Further, with respect to the predetermined time unit, the user's brain state is determined by using a brain activation change in each of a plurality of the divided brain areas S400.

### Method for stimulating brain

Meanwhile, prior to the step S100, a brain signal stimulating step may be additionally utilized.

FIG. 36 is a flowchart explaining a method for stimulating the user's brain for a signal collecting operation of the brain measuring portion.

Referring to FIG. 36, for the signal collecting operation of the brain signal measuring portion 10, the brain signal stimulating portion 20 stimulates the users' brain S50.

Then, the brain signal obtaining portion 11 of a brain signal measuring portion 10 irradiates near-infrared ray to the user's brain and detects light penetrating a cerebral cortex of the brain S100.

Meanwhile, the step S50 may include a step (a) in which a brain activating device of the brain signal stimulating portion applies multi-dimensional environmental stimulation to reinforce an activity of a pre-targeted brain area within a predetermined space intensively and to activate a function of the brain area; and a step (b) in which a management server controls an operation of the brain activating device to apply a multi-dimensional environmental stimulation to the pre-targeted brain area within the space.

Further, the space may be comparted into a plurality of spaces, and the step (a) may include: a step in which a media portion implements a media art corresponding to a concept to be created in each of the plurality of spaces; a step in which an acoustic portion outputs sound; a step in which a lighting portion irradiates light; and a step in which a scent transferring portion transfers a scent to the user.

Further, in the step (b), the management server may include database in which a plurality of media and acoustic information corresponding to a concept of each of the plurality of spaces and a program for controlling the operation of the brain activating device are stored, may select at least one of a plurality of media and acoustic information files and programs corresponding to the concept of each of the spaces from various kinds of the information and programs stored in the database, and may control the operation of the brain activating device allowing changing according to the selected information and program.

Further, the media portion may include a plurality of media modules which implement a media art corresponding to the concept of each of the spaces, each of the media modules including a projector or a display panel which projects the media art including a photo or a video, the acoustic portion including a plurality of acoustic modules which output sound corresponding to the concept of each of the spaces, and the each of the acoustic modules including a speaker and an amplifier which output sound including natural white noise.

Further, the lighting portion may include a plurality of lighting modules which irradiate light corresponding to the concept of each of the spaces, each of the lighting modules composed of a combination of one or two of a laser generator, an LED and a neon lighting, the scene transferring portion including a plurality of transferring modules which transfer scent generated from a natural object disposed in each of the spaces to the user, and each of the transferring modules including an air blower which blows air to transfer the scent generated from a natural object disposed in each of the spaces or a copy scent thereof to the user of each of the spaces.

Further, following the step (b), further included is a step (c) in which, on the basis of manager's operation, a manager' terminal that controls operation of the brain activating device selects at least one of the plurality of the information files and programs stored in the database of the management server for each of the modules to be installed in each of the spaces, and then changes media, sounds, lightings, etc., according to the selected information files and programs periodically or unperiodically.

Further, following the step (b), further included is a step (c) in which a user's terminal that is capable of communicating with the management server and inputs the user's operation instruction inputs the user's information including age, gender, preference and mental state from the user and then transfers the inputted user's information to the management server; and a step (d) in which the management server determines the user's age, gender, preference and mental state and performs controlling to provide media, sounds and lightings corresponding to a personalized concept.

### Method for determining brain state using body information related to user

According to another embodiment of the present invention, provided is a method for determining brain state using body information related to the user.

FIG. 37 is a flowchart explaining a method for determining the user's brain state by collecting body information related to the user and additionally using this information.

Referring to FIG. 37, the brain signal analyzing portion 13 of the brain signal measuring portion 10 extracts at least one brain activation area from a plurality of activated areas of the user's brain on the basis of the determined level of oxygenation of hemoglobin S300.

Wherein, prior to proceeding to the step S400, the user data collecting portion 20 collects body information related to the user S360.

Further, the diagnosing portion 50 determines a state of the user's brain on the basis of the at least one brain activation area extracted successively for a predetermined period of time together with the user's body information obtained from the step S360 S400.

Hereinafter, described are particular embodiments of the present invention regarding the step S360.

Firstly, FIG. 38 is a flowchart explaining a method for determining the user's brain state by determining a level of hearing damage and additionally using this level.

Referring to FIG. 38, the aforementioned step S400 may be composed of a step in which the diagnosing portion 50 determines a level of the user's hearing damage on the basis of the collected auditory information S410 and a step in which the diagnosing portion 50 determines the user's brain state using the at least one the brain activation area together with the level of user's hearing damage S420.

For example, when the user's hearing damage results from hearing loss, the diagnosing portion 50 determines the user's brain state by additionally using the body information related to the user, an average value of pure-tone thresholds in both of the user's ears, a level of the user's hearing loss and information related to the auditory information collecting portion; and

Further, when the user's hearing damage results from Tinnitus, the diagnosing portion 50 determines the user's brain state by additionally using the body information related to the user, an average value of pure-tone thresholds in both of the user's ears, a level of the user's hearing loss and information related to the auditory information collecting portion.

Further, in a case of this embodiment, a signal collecting operation of the brain signal obtaining portion 10 is performed in a state that the user's hearing damage occurs while the user is moving.

Further, FIG. 39 is a flowchart explaining a method for determining the user's brain state by additionally using a gait information collecting portion that collects the user's gait information collects the user's gait information, a stress collecting portion that collect the user's stress information, an electrocardiogram information collecting portion that collects the user's electrocardiogram information, a sleep information collecting portion that collects the user's sleep information and a attention information collecting portion that collects the user's attention information.

Referring to FIG. 39, the user data collecting portion 30 collects a gait information, the user's stress information, the user's electrocardiogram information, the user's sleep information, the user's attention information the user's electroencephalogram (EGG) information and the user's oxygen saturation S360.

Then, the diagnosing portion 50 determines, on the basis of the information collected from the user data collecting portion, at least one of a level of the user's hearing damage, the user's gait pattern, a level of the user's stress, the user's electrocardiogram change, sleep type, attention change, EGG change and oxygen saturation change S410.

The, the diagnosing portion 50 determines the user's brain state by using at least one of a level of the user's hearing damage, the user's gait pattern, a level of the user's stress, the user's electrocardiogram change, sleep type, attention change, EGG change and oxygen saturation change together with the at least one brain activation area S420.

In a case of this embodiment, the signal collecting operation of the brain signal measuring portion 10 may be performed under the circumstance being applied with at least one of states that the user's gait information is changing, the user's stress is changing, the user's electrocardiogram is changing, the user's sleep condition is changing and the user's attention is changing, while the user is moving.

Further, FIG. 40 is a flowchart explaining a method for determining the user's brain state by additionally using a non-contact typed emotional change collecting portion on the basis of eye tracking.

Referring to FIG. 40, the user data collecting portion 30 collects the user's face landmark masking data on the basis of the user's eye tracking to collect the user's emotional change information in a non-contact way S360.

Then, the diagnosing portion 50 determines the user's brain state using at least one brain activation area and the user's emotional change information together S420.

FIG. 41 is a flowchart explaining a method for determining the user's brain state by additionally using a non-contact typed emotional change collecting portion on the basis of speech change.

Referring to FIG. 41, the user data collecting portion 30 collects the user's emotional change information on the basis of the user's speech change S360.

Then, the diagnosing portion 50 determines the user's brain state using at least one brain activation area and the user's emotional change information together S420.

### Method for determining brain state using medical imaging related to user

Meanwhile, according to another embodiment of the present invention, provided is a method for determining a brain state using medical imaging related to the user.

FIG. 42 is a flowchart explaining a method for determining the user's brain state by additionally collecting and utilizing medical imaging related to the user.

Referring to FIG. 42, the brain signal analyzing portion 13 of the brain signal measuring portion 10 extracts at least one brain activation area from a plurality of activated areas of the user's brain on the basis of the determined level of oxygenation of hemoglobin S300.

Then, the user data collecting portion 30 collects body information related to the user S360. Prior to proceeding to the step S400, the imaging obtaining portion 40 collects medical imaging related to the user S370.

In the step S370, utilized are an MRI imaging scan obtaining portion that collects an MRI imaging scan related to the user; a CT imaging scan obtaining portion that collects a CT imaging scan related to the user; and an fMRI imaging scan obtaining portion that collects an fMRI imaging scan related to the user.

Then, the diagnosing portion 50 determines the user's brain state by using at least one brain activation area, the body information related to the user and the medical imaging related to the user together S400.

### Method for providing feedback corresponding to user's brain state

Further, according to the present invention, provided is a method for providing feedback corresponding to the user's brain state.

FIG. 43 FIG. 43 is a flowchart explaining method for providing information for improving the user's brain state corresponding to the user's brain state.

Referring to FIG. 43, the diagnosing portion 50 determines a state of the user's brain on the basis of the at least one brain activation area extracted successively for a predetermined period of time S400.

Then, the management portion 60 provides information for improving the user's brain state, corresponding to the user's brain state determined by the diagnosing portion 50 S500.

Regarding the step S500, FIG. 44 is a flowchart explaining a method for stimulating the brain in order to improve the user's brain state.

Referring to FIG. 44, by applying preset references, the user is suspected of at least one of dementia, MCI, Parkinson's disease, cerebral stroke, Epilepsy, brain tumor and development disorder within a certain period of time, on the basis of the user's brain state S410.

According thereto, the brain signal stimulating portion 20 stimulates the user's brain for improving the user's brain state according to controlling of the management portion S510.

Further, when the user's state falls into dementia, Parkinson's disease, cerebral stroke, Epilepsy, brain tumor and development disorder, it is prominent to record determination on a current state and to tract changes in those disorders, allowing checking and managing the user's brain state.

### Effects of the present invention

As described above, the present invention is capable of providing an AI based method for providing brain information which collects signals related with the brain of a user in a state of activity and determines the state of the user's brain therefrom and is capable of providing feedback on the brain information.

Further, the present invention is capable of providing a system and method for providing brain information, the system comprising: a brain signal measuring portion that includes a brain signal obtaining portion which irradiates near-infrared ray to user's brain and detects light penetrating the cerebral cortex of the brain, a brain signal processing portion which determines a level of oxygenation of hemoglobin in blood flow of the user's brain on the basis of the detected light and a brain signal analyzing portion which extract at least one brain activation area from a plurality of areas of the user's brain on the basis of the determined level of oxygenation of hemoglobin; and a diagnosing portion that determines the state of the user's brain on the basis of the at least one brain activation area which was extracted successively for a predetermined period of time.

Further, the present invention is capable of providing the system and method for providing brain information, the system including a brain signal stimulating portion for stimulating the user's brain.

Further, the present invention is capable of providing the system and method for providing brain information, the system determining the state of the user's brain more precisely by additionally using an auditory information collecting portion that collects the user's auditory information, a gait information collecting portion that collects the user's gait information, an electrocardiogram information collecting portion that collects the user's electrocardiogram information, a sleep information collecting portion that collects the user's sleep information, a attention information collecting portion that collects the user's attention information, a non-contact typed emotional change information collecting portion that collects the user's face landmark masking data on the basis of the user's eye tracking to collect the user's emotional change information in a non-contact way, a non-contact typed emotional change information collecting portion that collects the user's emotional change information on the basis of the user's speech change, etc.

Further, the present invention is capable of providing the system and method for providing brain information, the system determining the state of the user's brain more precisely by additionally using an imaging collecting portion that collects medical imaging related to the user and thus using the brain activation area, body information related to the user and the collected medical imaging related to the user together.

Further, the present invention is capable of providing the system and method for providing brain information, the system comprising a diagnosing portion that is capable of determining brain diseases including dementia, Parkinson's disease, cerebral stroke, Epilepsy, brain tumor and development disorder, and a management portion that provides information for improving brain state, corresponding to the user's brain state determined by the diagnosing portion.

Further, the present invention is capable of providing the system and method for providing brain information, the system further using a brain signal stimulating portion to stimulate the user's brain when the user, by applying preset references, is suspected of at least one of dementia, Parkinson's disease, cerebral stroke, Epilepsy, brain tumor and development disorder within a certain period of time, on the basis of the user's brain state.

Further, the present invention is capable of being used as a result of the study on degenerative brain diseases, thereby developing and utilizing a non-invasive diagnosis and treatment monitoring system through designing of brain imaging and cognitive ability measurement tests; preparing a base for building brain imaging database and sharing the database through brain structure imaging and brain function imaging tests; and providing the optimum treatment method through examination of a treatment mechanism based on deep-learning analysis.

Further, the present invention is capable of being used as a medical instrument for the optical treatment and diagnosis of degenerative brain disease, thereby implementing prototype of a medical instrument for commercialization of a treatment platform of the degenerative brain diseases; commercializing the medical instrument by cooperating with enterprises on the basis of the study result; and maximizing utilization of the medical instrument by liaison with local dementia care centers.

Furthermore, the present invention is capable of ensuring a high quality AI data set by preparing a standard guideline for each file of AI learning database constructions and verifying quality step by step; of providing basic data essential for AI technology development; and of building a virtuous cycle of ecosystem in private enterprises, which extends and opens the data autonomously according to AI technology development.

Further, the present invention is capable of leading the field of brain science by verifying treatment relation between the degenerative brain disease and hearing loss and of ensuring competitiveness in the field of advanced medical imaging analysis through studying deep-learning method of brain imaging data.

Further, the present invention is capable of ensuring a new distinctive technique for the non-invasive typed early diagnosis method of the degenerative brain disease and of developing a monitoring system of the non-invasive diagnosis and treatment of brain disease on the basis of brain imaging and cognitive ability.

Further, the present invention is capable of contributing to the solution of social problems through reducing dementia related deathrate, increasing cure rate and reducing treatment cost, and to the increment of productivity resulting from increment in the elderly people's social participation.

Further, the present invention is capable of identifying a state of brain health through the prevention and diagnosis of brain diseases when babies or children have the brain diseases including cerebral stroke, Epilepsy, brain tumor and development disorder or are suspected to have symptoms thereof.

Further, the present invention is capable of helping healthy brain development by increasing user's experience value and avoiding uses of sleeping drugs or anesthetic drugs as applying wearable typed and child friendly designed products when measuring brain signal pathway.

Further, the present invention is capable of providing, space for supporting non-contact learning and emotional labor fields, an emotion sharing AI service, that is, a human-centered AI service which utilizes the construction of a multimodal emotional data network and the N-dimensional emotion mapping.

In particular, the present invention is capable of recognizing user's social emotion; providing the construction of an emotional data network and a personalized emotional AI service; and being utilized for supporting non-contact learning and industrial work sites.

Meanwhile, the effects to be achieved by the present disclosure are not limited to the aforementioned effects and other effects, which are not mentioned above, will be apparent to those skilled in the art from the following description.

The aforementioned embodiments of the present invention may be implemented through various means. For example, the embodiments of the present invention may be implemented by hardware, software, firmware or any combination thereof.

When implemented by the hardware, the method cording to the embodiments of the present invention may be implemented by one or more selected from SICs(Application Specific Integrated Circuits), DSPs(Digital Signal Processors), DSPDs(Digital Signal Processing Devices), PLDs(Programmable Logic Devices), FPGAs(Field Programmable Gate Arrays), processors, controllers, micro-controllers, micro-processors, etc.

When implemented by the firmware or the software, the method cording to the embodiments of the present invention may be implemented into a form of modules, processes or functions which perform the aforementioned functions or operations. Software code may be operated by a processor stored in a memory unit. The memory unit is disposed in the interior or exterior of the processor and may exchange data with the processor by various means.

As aforementioned, those skilled in the art may implement the present invention referring to the detailed description of the embodiments. Even though the present invention is described referring to preferable embodiment above, it will be appreciated that those skilled in the art can correct and modify this invention variously within the scope thereof. For example, those skilled in the art are available to use each configurational element describe above in detail thorough a combination thereof. Accordingly, it is intended to grant the widest scope conforming to principals and novel characteristics disclosed herein rather than to limit the present invention to the embodiments disclosed herein.

The present invention may be implemented into other particular forms within the scope. Thus, the aforementioned detailed description should be considered as an example rather than understood limitedly. The scope of the present invention should be determined by comprehending the claimed inventions attached hereto. The scope of the present invention also includes all modification within a range equivalent thereto. Further, the present invention intends to grant the widest scope conforming to principals and novel characteristics disclosed herein rather than to limit the present invention to the embodiments disclosed herein. Further, it is allowable to configure an embodiment by combining claims which have no explicit citation relation with each other or to add new claims through amendments.

## Claims

1. An AI (Artificial Intelligence) based method for providing brain information comprising:
a step 1 in which a brain signal obtaining portion of a brain signal measuring portion irradiates near-infrared ray to user's brain and detects light penetrating a cerebral cortex of the brain;
a step 2 in which a brain signal processing portion of the brain signal measuring portion determines a level of oxygenation of hemoglobin in blood flow of the user's brain on the basis of the detected light;
a step 3 in which a brain signal analyzing portion of the brain signal measuring portion extracts at least one brain activation area from a plurality of activated areas of the user's brain on the basis of the determined level of oxygenation of hemoglobin; and
a step 4 in which a diagnosing portion determines a state of the user's brain on the basis of the at least one brain activation area extracted successively for a predetermined period of time, wherein
a signal collecting operation of the brain signal obtaining portion in the step 1 is performed in a state that the user is moving;
between the step 3 and the step 4, the method further comprises a step 3-5 in which a user data collecting portion collects body information related to the user, wherein
in the step 3-5,
the user data collecting portion comprises at least one of: an auditory information collection portion that collects the user's auditory information; a gait information collecting portion that collects the user's gait information collects the user's gait information; a stress collecting portion that collect the user's stress information; an electrocardiogram information collecting portion that collects the user's electrocardiogram information; a sleep information collecting portion that collects the user's sleep information; a attention information collecting portion that collects the user's attention information; a brainwave information collecting portion that collects the user's electroencephalogram (EGG) information; and an oxygen saturation information collecting portion that collects the user's oxygen saturation; and
in the step 4,
the diagnosing portion determines, on the basis of the collected auditory information, at least one of a level of the user's hearing damage, the user's gait pattern, a level of the user's stress, the user's electrocardiogram change, sleep type, attention change, EGG change and oxygen saturation change; and
determines the user's brain state using the at least one of the level of the user's hearing damage, the user's gait pattern, the level of the user's stress, the user's electrocardiogram change, sleep type, attention change, EGG change and oxygen saturation change together with the at least one brain activation area.

2. The AI (Artificial Intelligence) based method for providing brain information according to claim 1, wherein
in the step 2,
on the basis of the detected light, oxygenated hemoglobin (Oxy Hb) concentration and deoxygenated hemoglobin (Deoxy Hb) concentration in blood flow of the user are extracted to determine the level of oxygenation of hemoglobin;
in the at least one brain activation area,
oxygen transferred by the Oxy Hb in blood flow is consumed to decrease the Oxy Hb concentration and to increase the Deoxy Hb concentration corresponding to the decreased Oxy Hb concentration;
the Oxy Hb concentration and the Deoxy Hb concentration have an optical property that changes in a visible-ray area and a near-infrared area; and
in the steps 2, the brain signal measuring portion collects the signal through functional Near-infrared Spectroscopy (fNIRS).

3. The AI (Artificial Intelligence) based method for providing brain information according to claim 1, wherein
in the step 3, the brain signal analyzing portion divides the user's brain area into multiple areas and determines whether or not each of the divided brain areas is changed to the brain activation area by a predetermined time unit or whether or not the brain activation area is changed to an inactivation area.

4. The AI (Artificial Intelligence) based method for providing brain information according to claim 3, wherein
in the step 4,
the diagnosing portion, with respect to the predetermined time unit, uses a brain activation change in each of a plurality of the divided brain areas to determine the user's brain state.

5. The AI (Artificial Intelligence) based method for providing brain information according to claim 4, further comprising:
between the step 3 and the step 4,
a step in which a display portion of the brain signal measuring portion displays the brain activation change in each of the plurality of the divided brain areas according to the time.

6. The AI (Artificial Intelligence) based method for providing brain information according to claim 1, further comprising:
prior to the step 1,
a step 0.5 in which for a signal collecting operation of the brain signal measuring portion, a brain signal stimulating portion stimulates the users' brain.

7. The AI (Artificial Intelligence) based method for providing brain information according to claim 1, wherein
in the step 4, the diagnosing portion determines the user's brain state by using the at least one brain activation area together with a level of the user's hearing damage.

8. The AI (Artificial Intelligence) based method for providing brain information according to claim 7, wherein
when the user's hearing damage results from hearing loss,
the diagnosing portion determines the user's brain state by additionally using the body information related to the user, an average value of pure-tone thresholds in both of the user's ears, a level of the user's hearing loss and information related to the auditory information collecting portion; and
when the user's hearing damage results from Tinnitus,
the diagnosing portion determines the user's brain state by additionally using the body information related to the user, an average value of pure-tone thresholds in both of the user's ears, a level of the user's hearing loss and information related to the auditory information collecting portion.

9. The AI (Artificial Intelligence) based method for providing brain information according to claim 1, wherein
in the step 3-5, the user data collecting portion further comprises a non-contact typed emotional change information collecting portion that collects the user's face landmark masking data on the basis of the user's eye tracking to collect the user's emotional change information in a non-contact way, and
in the step 4, the diagnosing portion determines the user's brain state using the at least one brain activation area together with the user's emotional change information.

10. The AI (Artificial Intelligence) based method for providing brain information according to claim 1, wherein
in the step 3-5, the user data collecting portion further comprises a non-contact typed emotional change information collecting portion that collects the user's emotional change information on the basis of the user's speech change,
in the step 4, the diagnosing portion determines the user's brain state using the at least one brain activation area together with the user's emotional change information.

11. The AI (Artificial Intelligence) based method for providing brain information according to claim 1, further comprising:
between the step 3.5 and the step 4, a step 3.7 in which an imaging collecting portion collects medical imaging related to the user; wherein
in the step 4, the diagnosing portion determines the state of the user's brain using the at least one brain activation area, body information related to the user and the collected medical imaging related to the user together.

12. The AI (Artificial Intelligence) based method for providing brain information according to claim 11, wherein
the imaging collection portion of the step 3.7 comprises:
an MRI imaging scan obtaining portion that collects an MRI imaging scan related to the user; a CT imaging scan obtaining portion that collects a CT imaging scan related to the user; and an fMRI imaging scan obtaining portion that collects an fMRI imaging scan related to the user.

13. The AI (Artificial Intelligence) based method for providing brain information according to claim 1, wherein
in the step 4, the diagnosing portion determines the user's brain disease, the brain disease including dementia, mild cognitive impairment (MCI), Parkinson's disease, depression, cerebral stroke, Epilepsy, brain tumor and development disorder, and
following the step 4,
the method further comprises a step 5 in which a management portion provides information for improving the user's brain state, corresponding to the user's brain state determined by the diagnosing portion.

14. The AI (Artificial Intelligence) based method for providing brain information according to claim 13, wherein
when the user, by applying preset references, is suspected of at least one of dementia, MCI, Parkinson's disease, cerebral stroke, Epilepsy, brain tumor and development disorder within a certain period of time, on the basis of the user's brain state,
the step 5 further comprises a step in which a brain signal stimulating portion stimulates the user's brain for improving the user's brain state according to controlling of the management portion.

15. An AI (Artificial Intelligence) based device for providing brain information comprising:
a brain signal measuring portion that collects a signal related to user's brain;
a brain signal stimulating portion that stimulates the user's brain for an operation of collecting a signal of the brain signal measuring portion; and
a diagnosing portion that determines the user's brain state on the basis of the collected signal, wherein
the brain signal measuring portion comprises:
a brain signal obtaining portion that irradiates near-infrared ray to the user's brain and detects light penetrating a cerebral cortex of the brain;
a brain signal processing portion that determines a level of oxygenation of hemoglobin in blood flow of the user's brain on the basis of the detected light; and
a brain signal analyzing portion that extract at least one brain activation area which was activated from a plurality of areas of the user's brain on the basis of the determined level of oxygenation of hemoglobin,
the signal collecting operation of the brain signal measuring portion is performed in a state that the user is moving,
the diagnosing portion determines the user's brain state on the basis of the at least one brain activation area extracted successively for a predetermined period of time, and further comprises:
an imaging collecting portion that collects medical imaging related to the user; and
a user data collecting portion that collects body information related to the user,
the diagnosing portion determines the user's brain state by using the at least one brain activation area, the body information related to the user and the medical imaging related to the user together,
the diagnosing portion determines the user's brain disease, the brain disease including dementia, mild cognitive impairment (MCI), Parkinson's disease, depression, cerebral stroke, Epilepsy, brain tumor and development disorder, and further comprises:
a management portion that provides information for improving the user's brain state, corresponding to the user's brain state determined by the diagnosing portion,
the user data collecting portion comprises at least one of: an auditory information collection portion that collects the user's auditory information; a gait information collecting portion that collects the user's gait information collects the user's gait information; a stress collecting portion that collect the user's stress information; an electrocardiogram information collecting portion that collects the user's electrocardiogram information; a sleep information collecting portion that collects the user's sleep information; a attention information collecting portion that collects the user's attention information; a brainwave information collecting portion that collects the user's electroencephalogram (EGG) information; and an oxygen saturation information collecting portion that collects the user's oxygen saturation; and
the diagnosing portion determines, on the basis of the collected auditory information, at least one of a level of the user's hearing damage, the user's gait pattern, a level of the user's stress, the user's electrocardiogram change, sleep type, attention change, EGG change and oxygen saturation change, and
the diagnosing portion determines the user's brain state using the at least one of the level of the user's hearing damage, the user's gait pattern, the level of the user's stress, the user's electrocardiogram change, sleep type, attention change, EGG change and oxygen saturation change together with the at least one brain activation area.
